# FASCICULE DE BREVET EUROPEEN

(11) **EP 0 534 859 B1**
(45) Date de publication et mention de la délivrance du brevet: **17.11.1994**
(21) Numéro de dépôt: 92402630.5
(22) Date de dépôt: 25.09.1992
(51) Int. Cl.: C07D 223/16, C07D 405/12, C07D 491/056, A61K 31/55

(54) **3-Benzazépin-zones substituées par un groupe benzocyclobutyl- ou indanyl-alkyl-amino-alkyle, utiles dans le traitement des affections cardiovasculaires**
Benzocyclobutyl- oder Indanyl-Alkyl-Amino-Alkyl substituierte 3-Benzazepin-2-ones, verwendbar in der Behandlung von kardiovaskularen Krankheiten
Benzocyclobutyl- or indanyl-alkyl-amino-alkyl substituted 3-benzazepin-2-ones useful in the treatment of cardiovascular diseases

(30) Priorité: 27.09.1991 FR 9111894
(43) Date de publication de la demande: 31.03.1993
(73) Titulaire: ADIR ET COMPAGNIE, 92415 Courbevoie Cédex (FR)
(72) Inventeur: Peglion, Jean-Louis, F-78110 Le Vesinet (FR); Vian, Joel, F-92370 Chaville (FR); Vilaine, Jean-Paul, F-92290 Chatenay Malabry (FR); Villeneuve, Nicole, F-92500 Rueil Malmaison (FR); Janiak, Philip, F-92110 Clichy (FR); Bidouard, Jean-Pierre, F-92380 Chilly Mazarin (FR)
(74) Mandataire: Reverbori, Marcelle

(56) Documents cités:
- EP-A- 0 065 229
- EP-A- 0 161 604

## Description

La présente invention a pour objet de nouvelles (benzocycloalkyl) alkylamines, leur procédé de préparation, et les compositions pharmaceutiques qui les contiennent.

Elle concerne plus particulièrement les composés de formule générale (I) : 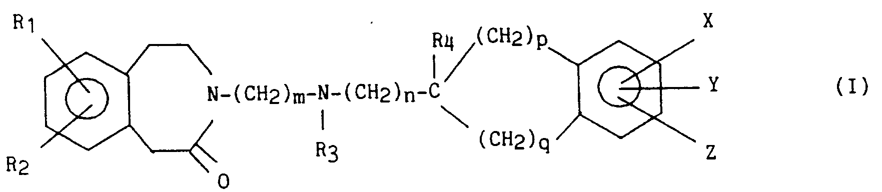
dans laquelle :
m représente un nombre entier de 2 à 5,
n représente un nombre entier de 1 à 6,
p et q, identiques ou différents, représentent 0 ou un nombre entier de 1 à 2,
à condition que la somme p + q soit égale à 1 ou 2,
R₁ et R₂, identiques ou différents, représentent un groupement choisi parmi :
- hydrogène,
- halogène,
- hydroxy,
- alcoxy inférieur,
- phénylalcoxy inférieur,
- et phénylalcoxy inférieur substitué,
   ou R₁ et R₂ forment ensemble, lorsqu'ils sont portés par 2 atomes de carbone adjacents, un groupement -O-(CH₂)ᵣ-O-avec r représentant un nombre entier égal à 1 ou 2,
R₃ représente un groupement choisi parmi :
- hydrogène,
- alkyle inférieur,
- alkényle inférieur,
- cycloalkyle,
- cycloalkyalkyle inférieur,
- phénylalkyle inférieur,
- phénylalkyle inférieur substitué,
- -CO-R₅ ou -CO-O-R₅
   avec R₅ signifiant un groupement choisi parmi : hydrogène, alkyle inférieur, alkényle inférieur, alkinyle inférieur, phényle, phényle substitué, phénylalkyle inférieur, phénylalkyle inférieur substitué, cycloalkyle inférieur, et cycloalkylalkyle inférieur,
- et -CO-NR₆R₇_{,} avec R₆ et R₇, identiques ou différents, ayant les mêmes significations que celles du groupement R₅ tel que défini ci-dessus,
   ou R₆ et R₇ forment ensemble, avec l'atome d'azote qui les porte, un cycle saturé de 4 à 7 chainons,
R₄ représente un atome d'hydrogène ou un radical alkyle inférieur ;
X, Y, Z, identiques ou différents, représentent un groupement choisi parmi :
- hydrogène,
- halogène,
- hydroxy,
- alcoxy inférieur,
- phénylalcoxy inférieur substitué,
   ou X et Y, ou Y et Z, forment ensemble, lorsqu'ils sont portés par 2 carbones adjacents, un groupement -O-(CH₂)ᵣ-O- dans lequel r représente un nombre entier égal à 1 ou 2, un groupement -O-(CH₂)₂- ou une groupement -O-CH=CH ;
étant entendu que le terme "substitué" affectant les groupements "phényle", "phénylalkyle inférieur" et "phénylalcoxy inférieur" signifie que ces groupements peuvent être substitués, sur le noyau phényle, par un ou plusieurs substituants choisis parmi : les atomes d'halogène, et les radicaux : hydroxy, trifluorométhyle, alkyle inférieur et alcoxy inférieur,
étant entendu - que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkinyle inférieur" désignent des groupements insaturés linéaires ou ramifiés contenant de 2 à 6 atomes de carbone,
- que le terme "cycloalkyle" désigne un cycle hydrocarboné saturé contenant de 3 à 8 chainons,
leurs éventuels isomères optiques, isolés ou sous forme de mélange, ainsi que, le cas échéant, leurs sels d'addition à un acide pharmaceutiquement acceptable.

L'art antérieur le plus proche est notamment illustré par des dérivés des 3-benzazépin 2-ones substituées par des groupements phénylalkylamine de formule (a) : 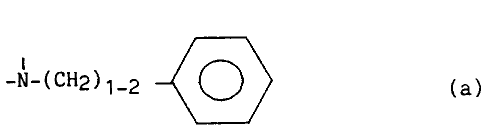
dans la demande **EP 0 065 229**,
ou par des groupements aminés des 1,2,3,4-tétrahydronaphtalènes de formule (b) : 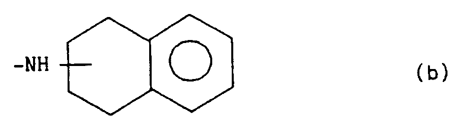
dans la demande **EP 0 161 604**.
Ces dérivés de l'art antérieur sont présentés dans les 2 demandes comme bradycardisants.

Les composés de la présente invention se distinguent de ceux de l'art antérieur par la présence, à la place des groupements (a) et (b) précédemment définis, de groupements (benzocyclobut-1 yl)alkylamine et (indan-2 yl)alkylamine.La découverte par la demanderesse de puissantes activités bradycardisantes, mais également anti-arythmiques et anti-ischémiantes, de longue durée d'action, est donc surprenante puisque de tels groupements (benzocyclobut-1 yl)alkylamine et (indan-2 yl)alkylamines remplacent très avantageusement les groupements de formule (a) et (b) de l'art antérieur.

Ces différences de structures conduisent en outre à des composés se distinguant de ceux de l'art antérieur par une meilleure sélectivité et une durée d'action supérieure.

L'invention s'étend également au procédé de préparation des composés de formule (I), caractérisé en ce que :

on condense une amine de formule (II) : 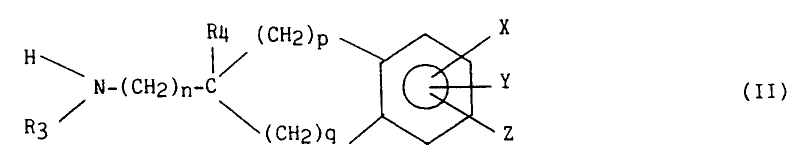
dans laquelle R₃, R₄, X, Y, Z, n,p et q sont tels que définis dans la formule (I), sous forme racémique ou optiquement active lorsqu'il existe un carbone asymétrique,
avec un composé de formule (III) : 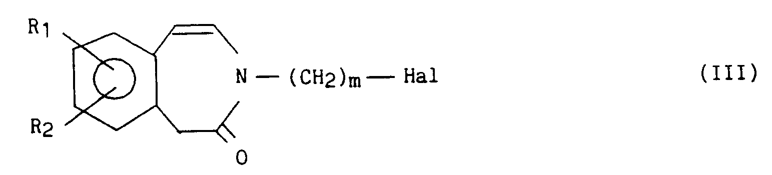
dans laquelle R₁, R₂, et m sont tels que définis dans la formule (I), et Hal représente un atome d'halogène,
pour obtenir un composé de formule (IV) : 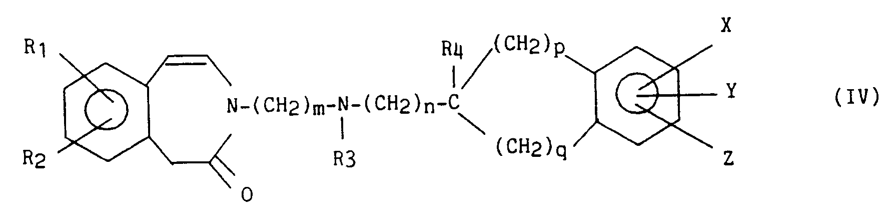
dans laquelle R₁, R₂, R₃, R₄, X, Y, Z, m, n, p et q sont tels que définis précédemment, sous forme racémique ou optiquement active lorsqu'il existe un carbone asymétrique,
composé de formule IV qui est soumis à une hydrogénation afin d'obtenir un composé de formule (I) : 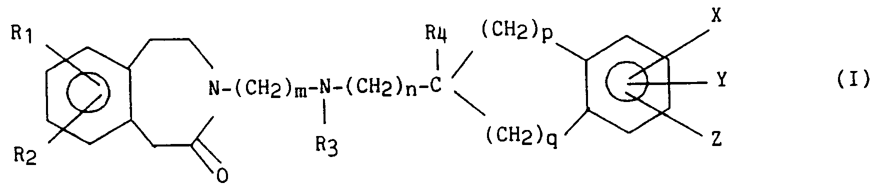
dans laquelle R₁, R₂, R₃, R₄, X, Y, Z, m, n, p et q sont tels que définis précédemment, sous forme racémique ou optiquement active lorsqu'il existe un carbone asymétrique,
lequel composé de formule (I) est, si on le désire, :
- purifié par une technique classique de cristallisation et/ou de chromatographie,
- et/ou salifié avec un acide pharmaceutiquement acceptable,
étant entendu que les composés de formule (I), lorsqu'ils possèdent un carbone asymétrique peuvent être préparés sous une forme optiquement active non seulement en utilisant des matières premières optiquement actives mais également a' partir des composés racémiques de formule (I) correspondants, par des méthodes classiques de séparation des isomères optiques.

Les composés de formule (IV) sont nouveaux et font partie de l'invention au même titre que les composés de formule (I) dont ils constituent les intermédiaires de synthèse.

Les composés de formule générale (I) peuvent être transformés en sels d'addition avec les acides, sels qui font à ce titre, partie de l'invention. Comme acides utilisables pour la formation de ces sels, on peut citer, par exemple, dans la série minérale les acides chlorhydrique, bromhydrique, sulfurique, phosphorique et dans la série organique, les acides acétique, propionique, maléique, fumarique, tartrique, nitrique, oxalique, benzoïque, méthanesulfonique, iséthionique, benzenesulfonique,..

Les propriétés pharmacologiques des produits de la présente invention révèlent leur intérêt en thérapeutique cardiovasculaire.

Les études réalisées in vivo montrent leur activité spécifique puissante et de longue durée d'action, réduisant la fréquence cardiaque et permettant de diminuer la consommation d'oxygène myocardique.

Les études réalisées confirment que l'activité de ces composés est directe sur le noeud sinusal et se distingue de celle des antagonistes des récepteurs Béta-adrénergiques et des inhibiteurs des canaux calciques, notamment par l'absence d'effets dépresseurs sur
la conduction auriculo-ventriculaire et sur la fonction contractile cardiaque.

Ces propriétés permettent l'utilisation des dérivés de l'invention, comme médicaments à titre curatif ou préventif des différentes situations cliniques d'ischémie myocardique qui résultent d'un déséquilibre entre l'apport et la demande en oxygène myocardique telles que l'angine de poitrine, l'infarctus du myocarde et les troubles du rythme associés, ainsi que dans les différentes pathologies comportant des troubles du rythme, notamment supra-ventriculaires.

Ces produits peuvent également réduire les complications des lésions athéroscléreuses notamment coronaires par limitation des contraintes hémodynamiques vasculaires.

La présente invention a également pour objet les compositions pharmaceutiques contenant les produits de formule (I) ou un de leurs sels d'addition à un acide pharmaceutiquement acceptable, seuls ou en combinaison avec un ou plusieurs excipients ou véhicules inertes non toxiques, pharmaceutiquement acceptables.

Les compositions pharmaceutiques ainsi obtenues se présentent généralement sous forme dosée. Elles peuvent par exemple revêtir la forme de comprimés, dragées, gélules, suppositoires, solutions injectables ou buvables et être administrées par la voie orale, rectale, intramusculaire, ou parentérale.

La posologie peut varier notablement selon l'âge et le poids du patient, la voie d'administration, la nature de la maladie et les traitements associés et consiste en prises de 1 à 100 mg, une à plusieurs fois par jour.

Les exemples suivants illustrent l'invention mais ne la limitent en aucune façon.

Les points de fusion sont déterminés à la platine chauffante Kofler. Les spectres de résonnance magnétique nucléaire 1H (RMN) ont été réalisés en utilisant le tétraméthylsilane (TMS) comme référence interne. Les déplacements chimiques sont exprimés en partie par million (ppm).

Les matières premières utilisées dans les exemples suivants sont soit des produits connus, soit des produits préparés à partir de substances connues selon des procédés décrits pour préparer des produits analogues.

### EXEMPLE 1

### (R,S) 7,8-diméthoxy 3-{3-{N-[(4,5-diméthoxy benzocyclobut-1 yl)méthyl] N-(méthyl) amino}propyl} 1,3,4,5-tétrahydro 2H 3-benzazépin 2-one

### STADE A : (R,S) N-[(4,5-diméthoxy benzocyclobut-1 yl)méthyl] N-(méthyl)amine

### ETAPE A₁ :

### Chlorhydrate de (R,S) N-[(4,5-diméthoxy benzocyclobut-1 yl)méthyl]amine

On ajoute, goutte à goutte et sous agitation à température ambiante, 312 cm³ d'une solution molaire de borane complexé avec du tétrahydrofurane à une solution de 25 g de (R,S) 1-(cyano) 4,5-(diméthoxy) benzocyclobutane dans 250 cm³ de tétrahydrofurane. On laisse en contact pendant 12 heures, puis on ajoute 200 cm³ d'éthanol, et on agite 1 heure. On ajoute, goutte à goutte, 100 cm³ d'éther chlorhydrique 3,3 N. On obtient 27,7 g du composé attendu.
Rendement : 90 %
Point de fusion : 205 °C.

### ETAPE A₂ :

### (R,S) N-[(4,5-diméthoxy benzocyclobut-1 yl)méthyl] N-(éthoxycarbonyl)amine

On coule 1,5 cm³ de chlorofomiate d'éthyle sur une suspension composée de 3,4 g du composé obtenu à l'étape A₁, de 4,5 cm³ de triéthylamine et de 50 cm³ de dichlorométhane.
On laisse une nuit sous agitation à température ambiante puis on lave à l'eau, et à l'acide chlorhydrique 1N. On sèche et on évapore à sec le solvant. On obtient 3,2 g d'une huile correspondant au composé attendu.
Rendement : 80 %.

### ETAPE A₃ :

### (R,S) N-[(4,5-diméthoxy benzocyclobut-1 yl)méthyl] N-(méthyl)amine

On ajoute 3,2 g du composé obtenu à l'étape A₂ en solution dans 30 cm³ de tétrahydrofurane à une suspension de 0,9 g d'hydrure de lithium et d'aluminium dans 20 cm³ de tétrahydrofurane. On porte au reflux 1 heure 30 minutes puis on hydrolyse par 0,6 cm³ d'eau puis 0,5 cm³ de soude à 20 %, et enfin par 2,3 cm³ d'eau.
Les sels minéraux sont ensuite filtrés, rincés au tétrahydrofurane puis le filtrat obtenu est évaporé à sec. On obtient 2,3 g du composé du stade A.
Rendement : 92 %.

### STADE B : 7,8-diméthoxy 3-[3-(iodo)propyl] 1,3-dihydro 2H 3-benzazépin 2-one

On porte au reflux pendant 24 heures une suspension de 12 g de 7,8-diméthoxy 3-[3-(chloro)propyl] 1,3-dihydro 2H 3-benzazépin 2-one obtenue selon la méthode décrite dans la littérature (Reiffer M. et al., J. Med Chem 1990 ; vol 33 (5) : 1496-1504) et de 6,2 g d'iodure de sodium dans 50 cm³ d'acétone.
Après filtration et évaporation à sec du solvant, le résidu est repris à l'eau et extrait au dichlorométhane. On décante, on sèche sur sulfate de magnésium anhydre, puis on concentre sous vide pour obtenir 15,2 g du composé désiré.
Rendement : 99 %.
Point de fusion : 132-134 °C.

| Caractéristiques sprectrales : RMN (CDCl₃) | | | | | |
|---|---|---|---|---|---|
| 6,8 ppm | 2 singulets | 2H | 6,35-6,2 ppm | 2 doublets | 2H |
| 3,9 ppm | 2 singulets | 6H | 3,6 ppm | triplet | 2H |
| 3,45 ppm | multiplet | 2H | 3,1 ppm | triplet | 2H |
| 2,1 ppm | multiplet | 2H | | | |

### STADE C : (R,S) 7,8-diméthoxy 3-{3-{N-[(4,5-diméthoxy benzocyclobut-1 yl)méthyl] N-(méthyl)amino}propyl} 1,3-dihydro 2H 3-benzazépin 2-one

On porte au reflux, pendant 18 heures, un mélange composé de 5,6 g de carbonate de potassium, de 2,2 g du composé obtenu au stade A dans 100 cm³ d'acétone, et de 4 g du composé obtenu au stade B.

On évapore le solvant sous vide, on reprend le résidu à l'acétate d'éthyle, puis on extrait à l'acide chlorhydrique 3N.
On basifie la phase acide décantée à l'aide d'hydroxyde de sodium, puis on l'extrait à l'acétate d'éthyle. Après lavage à neutralité, et séchage sur sulfate de magnésium anhydre, on évapore sous vide pour obtenir 4,5 g d'une huile qui est purifiée sur colonne de silice en utilisant comme éluant un mélange chlorure de méthylène/méthanol (90/10, v/v).
Rendement : 64 %.

| Caractéristiques spectrales : RMN (CDCl₃) | | | | | |
|---|---|---|---|---|---|
| 6,7 ppm | singulet | 4H | 6,8 et 6,65 ppm | 2 doublets | 2H |
| 3,85 ppm | singulet | 12H | 3,65 ppm | multiplet | 3H |
| 3,45 ppm | singulet | 2H | 3,2 ppm | doublet | 1H |
| 2,7-2,1 ppm | multiplet | 5H | 2,25 ppm | singulet | 3H |
| 1,7 ppm | singulet | 2H | | | |

### STADE D : (R,S) 7,8-diméthoxy 3-{3-{N-[(4,5-diméthoxy benzocyclobut-1 yl)méthyl] N-(méthyl)amino}propyl} 1,3,4,5-tétrahydro 2H 3-benzazépin 2-one

5 g du composé obtenu au stade C dans 50 cm³ d'acide acétique glacial sont hydrogénés dans un appareil de Parr, sous une pression de 49.10⁴ Pa d'hydrogène à température ambiante pendant 24 heures, en présence de 1 g d'hydroxyde de palladium à 10 %. On filtre le catalyseur, on évapore le solvant, puis on reprend le résidu sec à l'eau et à l'acétate d'éthyle. On basifie la phase aqueuse décantée à l'hydroxyde de sodium, puis on l'extrait à l'acétate d'éthyle. On sèche la phase organique sur sulfate de magnésium anhydre, on concentre sous vide ; puis le résidu est purifié sur colonne de silice en utilisant comme éluant un mélange chlorure de méthylène/méthanol (95/5,v/v).
Après recristallisation de l'acétate d'éthyle, on obtient 2 g du composé de l'exemple.
Rendement : 40 %.
Point de fusion : 101-103 °C.

| Caractéristiques spectrales : RMN (CDCl₃) | | |
|---|---|---|
| 6,7 ppm | 2 singulets | 2H |
| 6,55 ppm | 2 singulets | 2H |
| 3,9-3,6 ppm | 2 singulets et 1 triplet | 16H |
| 3,5 ppm | multiplet | 3H |
| 3,25 ppm | doublet | 1H |
| 3,05 ppm | triplet | 2H |
| 2,8-2,3 ppm | doublet, multiplet et triplet | 5H |
| 2,3 ppm | singulet | 3H |
| 1,75 ppm | multiplet | 2H |

### EXEMPLE 2

### (+) 7,8-diméthoxy 3-{3-{N-[(4,5-diméthoxybenzocyclobut-1 yl)méthyl] N-(méthyl)amino}propyl} 1,3,4,5-tétrahydro 2H 3-benzazépin 2-one

En procédant comme dans l'exemple 1, mais en utilisant la l'orme optiquement active (+), dédoublée sous forme de sel de (d) camphosulfonate, du composé obtenu au stade A de l'exemple 1, on obtient :

### STADE A : (+) N-[(4,5-diméthoxy benzocyclobut-1 yl)méthyl] N-(méthyl)amine

On fait réagir la (R,S) N-[(4,5-diméthoxy benzocyclobut-1 yl)méthyl] N-(méthyl)amine obtenue au stade A de l'exemple 1 avec une quantité équimolaire de l'acide (d) camphosulfonique dans l'éthanol.
Après évaporation sous vide du solvant, le sel est recristallisé une première fois dans l'acétate d'éthyle puis dans l'acétonitrile jusqu'à l'obtention de l'énantiomère (+) avec une pureté optique supérieure à 99 % (évaluation par Chromatographie Liquide Haute Performance sur colonne CHIRALCEL®OD).
Point de fusion ((d) camphosulfonate) : 160-162 °C.
Pouvoir rotatoire (concentration : 1 % dans le DMSO).

| λ nm | [α] 20,5 °C |
|---|---|
| 589 | + 32,0 ° |
| 578 | + 33,7 ° |
| 546 | + 39,7 ° |
| 436 | + 83,9 ° |
| 365 | + 195,6 ° |

### STADE B : 7,8-diméthoxy 3-[3-(iodo)propyl] 1,3-dihydro 2H 3-benzazépin 2-one

### STADE C : (+) 7,8-diméthoxy 3-{3-{N-[(4,5-diméthoxy benzocyclobut-1 yl)méthyl] N-(méthyl)amino}propyl} 1,3-dihydro 2H 3-benzazépin 2-one.

Le sel de (d) camphosulfonate du composé obtenu au stade A en solution dans l'acétate d'éthyle est préalablement basifié à l'aide d'hydroxyde de sodium, puis la phase organique est séparée, lavée, séchée sur sulfate de sodium anhydre, et évaporée avant d'être mise en réaction selon le stade C de l'exemple 1.

### STADE D : Dibenzoyltartrate de l'isomère (+) du 7,8-diméthoxy 3-{3-{N-[(4,5-diméthoxy benzocyclobut-1 yl)méthyl] N-(méthyl)amino}propyl} 1,3,4,5-tétrahydro 2H 3-benzazépin 2-one.

Point de fusion : 104-106 °C.
Solvant de recristallisation du sel de dibenzoyltartrate : H₂O.
Pouvoir rotatoire (base, concentration : 1 % dans le CHCl₃).

| λ nm | [α] 20,5 °C |
|---|---|
| 589 | + 3,9 ° |
| 578 | + 4,4 ° |
| 546 | + 4,9 ° |
| 436 | + 8,10 ° |
| 365 | énergie insuffisante |

**STADE E** : Monochlorhydrate de l'isomère (+) du 7,8-diméthoxy 3-{3-{N-[(4,5-diméthoxy benzocyclobut-1 yl)méthyl] N-(méthylamino}propyl} 1,3,4,5-tétrahydro 2H 3-benzazépin 2-one.

A 0,7 g de (+) 7,8-diméthoxy 3-{3-{N-[(4,5-diméthoxy benzocyclobut-1 yl)méthyl] N-(méthylamino}propyl} 1,3,4,5-tétrahydro 2H 3-benzazépin 2-one, on ajoute 14,9 ml d'HCl 0,1 N. On agite, filtre, concentre et recristallise dans 5 ml d'acétonitrile. On obtient 0,5 g de monochlorhydrate correspondant (rendement : 66 %).
Point de fusion (instantané) : 135-140 °C.
Pouvoir rotatoire (1 % dans le DMSO)

| λ nm | [α] 21 °C |
|---|---|
| 589 | + 7,8 ° |
| 578 | + 8,0 ° |
| 546 | + 9,0 ° |
| 436 | + 15,3 ° |
| 365 | + 27,8 ° |

### EXEMPLE 3

### (-) 7,8-diméthoxy 3-{3-{N-[(4,5-diméthoxybenzocyclobut-1 yl)méthyl] N-(méthyl)amino}propyl} 1,3,4,5-tétrahydro 2H 3-benzazépin 2-one.

De façon anologue à l'exemple 2, mais en utilisant la forme optiquement active (-), dédoublée sous forme de sel de (l)-camphosulfonate, du composé obtenu au stade A de l'exemple 1, on obtient :

### STADE A : (-) N-[(4,5-diméthoxy benzocyclobut-1 yl)méthyl] N-(méthyl)amine

Point de fusion ((l) camphosulfonate) : 160-162 °C.
Pouvoir rotatoire (concentration : 0,85 % dans le DMSO).

| λ nm | [α] 20,5 °C |
|---|---|
| 589 | - 32,2 ° |
| 578 | - 34,1 ° |
| 546 | - 39,9 ° |
| 436 | - 84,5 ° |
| 365 | - 198,1 ° |

### STADE B : 7,8-diméthoxy 3-[3-(iodo)propyl] 1,3-dihydro 2H 3-benzazépin 2-one

### STADE C : (-) 7,8-diméthoxy 3-{3-{N-[(4,5-diméthoxy benzocyclobut-1 yl)méthyl] N-(méthyl)amino}propyl} 1,3-dihydro 2H 3-benzazépin 2-one

### STADE D : Dibenzoyltartrate de l'isomère (-) du 7,8-diméthoxy 3-{3-{N-[(4,5-diméthoxy benzocyclobut-1 yl)méthyl] N-(méthyl)amino}propyl} 1,3,4,5-tétrahydro 2H 3-benzazépin 2-one.

Point de fusion (dibenzoyltartrate) : 104-106 °C.
Solvant de recristallisation du sel de dibenzoyltartrate : H₂O.
Pouvoir rotatoire (concentration : 0,85 % dans le DMSO).

| λ nm | [α] 20,5 °C |
|---|---|
| 589 | - 4,3 ° |
| 578 | - 4,6 ° |
| 546 | - 5,0 ° |
| 436 | - 8,50 ° |
| 365 | énergie insuffisante |

### STADE E : Monochlorhydrate de l'isomère (-) du 7,8-diméthoxy 3-{3-{N-[(4,5-diméthoxy benzocyclobut-1 yl)méthyl] N-(méthylamino}propyl} 1,3,4,5-tétrahydro 2H 3-benzazépin 2-one.

En opérant comme dans l'exemple 2 stade E, on obtient avec un rendement de 50 % le monochlorhydrate attendu.
Point de fusion (instantané) : 135-140 °C.
Pouvoir rotatoire ( 1 % dans le DMSO).

| λ nm | [α] 21 °C |
|---|---|
| 589 | - 6,0 ° |
| 578 | - 6,2 ° |
| 546 | - 7,2 ° |
| 436 | - 13,5 ° |
| 365 | - 26,5 ° |

### EXEMPLE 4

### (R,S) 7,8-diméthoxy 3-{3-{N-[(4,5-diméthoxy benzocyclobut-1 yl)méthyl]amino} propyl} 1,3,4,5-tétrahydro 2H 3-benzazépin 2-one

En procédant comme dans l'exemple 1, mais en remplaçant à l'étape A₂ du stade A le chloroformiate d'éthyle par le chlorure de benzoyle, on obtient successivement :

### STADE A : (R,S) N-{[4,5-diméthoxy benzocyclobut-1 yl)méthyl} N-(benzyl)amine

Rendement : 90,4 %.

### ETAPE A₁

Chlorhydrate de (R,S) N-[(4,5-diméthoxy benzocyclobut-1 yl)méthyl]amine

### ETAPE A₂

(R,S) N-{[4,5-diméthoxy benzocyclobut-1 yl]méthyl} N-(benzoyl)amine.
Rendement : 98 %
Point de fusion : 142-144 °C.

### STADE B : 7,8-diméthoxy 3-[3-(iodo)propyl] 1,3-dihydro 2H 3-benzazépin 2-one.

### STADE C : (R,S) 7,8-diméthoxy 3-{3-{N-[(4,5-diméthoxy benzocyclobut-1 yl)méthyl] N-(benzyl)amino}propyl} 1,3-dihydro 2H 3-benzazépin 2-one

Rendement : 62 %.

### STADE D : (R,S) 7,8-diméthoxy 3-{3-{N-[(4,5-diméthoxy benzocyclobut-1 yl)méthyl]amino}propyl} 1,3,4,5-tétrahydro 2H 3-benzazépin 2-one

Point de fusion (dichlorhydrate) : 185-188 °C.
Solvant de recristallisation du sel de dichlorhydrate : acétate d'éthyle.

### EXEMPLE 5

### (R,S) 7,8-diméthoxy 3-{3-{N-[2-(4,5-diméthoxy benzocyclobut-1 yl)éthyl]amino} propyl} 1,3,4,5-tétrahydro 2H 3-benzazépin 2-one

En procédant comme dans l'exemple 1, mais en remplaçant à l'étape A₁ le (R,S) 1-(cyano) 4,5-(diméthoxy) benzocyclobutane par le (R,S) (4,5-diméthoxy benzocyclobut-1 yl) acétonitrile et à l'étape A₂ le chloroformiate d'éthyle par le chlorure de benzoyle, on obtient :

### STADE A : (R,S) N-[2-(4,5-diméthoxy benzocyclobut-1 yl)éthyl] N-(benzyl)amine

Rendement : 70 %.

### ETAPE A₁

Chlorhydrate de (R,S) N-[2-(4,5-diméthoxy benzocyclobut-1 yl)éthyl]amine.
Rendement : 41 %

### ETAPE A₂

(R,S) N-[2-(4,5-diméthoxy benzocyclobut-1 yl)éthyl]N-(benzoyl)amine
Rendement : 98 %.

### STADE B : 7,8-diméthoxy 3-[3-(iodo)propyl] 1,3-dihydro 2H 3-benzazépin 2-one

### STADE C : (R,S) 7,8-diméthoxy 3-{3-{N-[2-(4,5-diméthoxy benzocyclobut-1 yl) éthyl] N-(benzyl)amino}propyl} 1,3-dihydro 2H 3-benzazépin 2-one

Rendement : 48 %.

### STADE D : (R,S) 7,8-diméthoxy 3-{3-{N-[2-(4,5-diméthoxy benzocyclobut-1 yl) éthyl] amino}propyl 1,3,4,5-tétrahydro 2H benzazépin 2-one.

Point de fusion (acétate) : 98-102 °C.
Solvant de recristallisation du sel d'acétate : éther isopropylique.

### EXEMPLE 6

### (R,S) 7,8-diméthoxy 3-{3-{N-[2-(4,5-diméthoxy benzocyclobut-1 yl)éthyl N-(méthyl)amino}propyl} 1,3,4,5-tétrahydro 2H 3-benzazépin 2-one

En procédant comme dans l'exemple 1, mais en remplaçant lors de l'étape A₁ le (R,S) 1-(cyano) 4,5-(diméthoxy) benzocyclobutane par le (R,S) (4,5-diméthoxy benzocyclobut-1 yl) acétonitrile, on obtient successivement :

### STADE A : (R,S) N-[2-(4,5-diméthoxy benzocyclobut-1 yl)éthyl] N-(méthyl)amine

Rendement : 68 %.

### ETAPE A₁ : chlorhydrate de (R,S) N-[2-(4,5-diméthoxy benzocyclobut-1 yl)éthyl] amine

### ETAPE A₂ : (R,S) N-[2-(4,5-diméthoxy benzocyclobut-1 yl)éthyl] N-(éthoxycarbonyl) amine.

Rendement : 98 %.

### STADE B : 7,8-diméthoxy 3-[3-(iodo)propyl] 1,3-dihydro 2H 3-benzazépin 2-one

### STADE C : (R,S) 7,8-diméthoxy 3-{3-{N-[2-(4,5-diméthoxy benzocyclobut-1 yl) éthyl] N-(méthyl)amino}propyl} 1,3-dihydro 2H 3-benzazépine 2-one

Rendement : 62 %.

### STADE D : (R,S) 7,8-diméthoxy 3-{3-{N-[2-(4,5-diméthoxy benzocyclobut-1 yl) éthyl] N-(méthyl)amino}propy} 1,3,4,5-tétrahydro 2H 3-benzazépin 2-one

Point de fusion (dichlorhydrate) : 102-105 °C.
Solvant de recristallisation du sel de dichlorhydrate: éther.

### EXEMPLE 7

### (R,S) 7,8-diméthoxy 3-{3-{N-[(5-méthoxy benzocyclobut-1 yl)méthyl] N-(méthyl)amino}propyl} 1,3,4,5-tétrahydro 2H 3-benzazépin 2-one

En procédant comme dans l'exemple 1, mais en remplaçant à l'étape A₁ le (R,S) 1-(cyano) 4,5-(diméthoxy) benzocyclobutane par le (R,S) 1-(cyano) 5-(méthoxy) benzocyclobutane, on obtient aux deux derniers stades :

### STADE C : (R,S) 7,8-diméthoxy 3-{3-{N-[(5-méthoxy benzocyclobut-1 yl) méthyl] N-(méthyl)amino}propyl} 1,3-dihydro 2H 3-benzazépin 2-one.

### STADE D : (R,S) 7,8-diméthoxy 3-{3-{N-[(5-méthoxy benzocyclobut-1 yl)méthyl] N-(méthyl)amino}propyl} 1,3,4,5-tétrahydro 2H 3-benzazépin 2-one.

Rendement : 48 %
Point de fusion (dichlorhydrate) : 125-130 °C.
Solvant de recristallisation du sel de dichlorhydrate : acétate d'éthyle.

### EXEMPLE 8

### (R,S) 7,8-diméthoxy 3-{3-[(benzocyolobut-1 yl)méthyl] N-(méthyl)amino}propyl} 1,3,4,5-tétrahydro 2H 3-benzazépin 2-one

En procédant comme dans l'exemple 1, mais en remplaçant à l'étape A1 le (R,S) 1-(cyano) 4,5-(diméthoxy) benzocyclobutane par le (R,S) 1-(cyano) benzocyclobutane, on obtient aux deux derniers stades :

### STADE C : (R,S) 7,8-diméthoxy 3-{3-[(benzocyclobut-1 yl)méthyl] N-(méthyl)amino}propyl} 1,3-dihydro 2H 3-benzazépin 2-one.

### STADE D : (R,S) 7,8-diméthoxy 3-{3-[benzocyclobut-1 yl)méthyl] N-(méthyl)amino}propyl} 1,3,4,5-tétrahydro 2H 3-benzazépin 2-one.

Rendement : 45 %
Point de fusion (dichlorhydrate) : 128-132 °C.
Solvant de recristallisation du sel de dichlorhydrate : acétate d'éthyle

### EXEMPLE 9

### 7,8-diméthoxy 3-{3-{N-[(5,6-diméthoxyindan-2 yl)méthyl] N-(méthyl)amino} propyl} 1,3,4,5-tétrahydro 2H 3-benzazépin 2-one.

### STADE A : N-[(5,6-diméthoxy indan-2 yl)méthyl] N-(méthyl)amine.

### ETAPE A1 : 5,6-(diméthoxy) 2-(éthoxycarbonyl) indan 1-one.

On ajoute à une suspension de 20 g d'hydrure de sodium à 50 % (préalablement lavée à l'hexane) dans 240 cm³ de tétrahydrofurane, 50,5 cm³ de carbonate de diéthyle.
On porte au reflux pendant 1 heure 30 minutes, puis on ajoute une solution de 40 g de 5,6-diméthoxy indan 1-one dans 440 cm³ de tétrahydrofurane à température ambiante.
On porte de nouveau au reflux pendant 3 heures, puis le milieu réactionnel est refroidi, dilué en présence d'acétate d'éthyle, et traité par une solution aqueuse d'acide acétique.
On décante la phase organique, on la sèche sur sulfate de magnésium anhydre, puis on la concentre sous vide pour obtenir 52 g du composé désire.
Rendement : 98 %.
Point de fusion : 132-134 °C.

### ETAPE A₂

### Acide (5,6-diméthoxy indan) 2-oïque.

On ajoute, sous agitation, 117 g de zinc et 11,7 g de chlorure mercurique à un mélange composé de 195 cm³ d'acide chlorhydrique concentré dans 105 cm³ d'eau et de 52 g du composé obtenu à l'étape A₁ dans 520 cm³ de toluène.
On porte le milieu réactionnel au reflux pendant 24 heures. On refroidit et on décante la phase toluénique puis on l'épuise à l'aide d'une solution d'hydroxyde de sodium 1N.
Après décantation, on acidifie la phase aqueuse par de l'acide chlorhydrique 1N. On l'extrait ensuite à l'acétate d'éthyle, puis on décante la phase organique, on la sèche sur sulfate de magnésium anhydre. On filtre et on concentre sous vide pour obtenir 20 g du composé attendu.
On évapore sous vide la phase toluénique précédente pour recueillir 19 g de 5,6-diméthoxy 2-éthoxycarbonyl indane, que l'on met à réagir sous agitation, pendant 18 heures et à température ambiante, en présence de 80 cm³ d'éthanol et de 80 cm³ d'hydroxyde de sodium 1N.
On évapore à sec, puis on acidifie à l'acide chlorhydrique 1N, on extrait à l'acétate d'éthyle et on concentre sous vide pour donner 14,8 g du composé désiré qui sont réunis aux 20 g précédemment obtenus.
Rendement : 78 %.
Point de fusion : 126-128 °C.

### ETAPE A₃

### N-[(5,6-diméthoxy indan-2 yl)carbonyl] N-(méthyl)amine.

On ajoute par portions, à une solution de 14 g du composé obtenu à l'étape A₂ dans 150 cm³ de chlorure de méthylène, 10,2 g de carbonyldiimidazole, et on agite pendant 4 heures.
On sature la solution par de la méthylamine pendant 4 heures, sous agitation, à température ambiante. On dilue le mélange réactionnel avec de l'eau. On décante, on lave à l'hydroxyde de sodium 1N, on décante à nouveau, on sèche sur sulfate de magnésium anhydre, et on évapore à sec. On obtient ainsi le composé attendu qui est recristallisé dans l'éthanol.
Point de fusion : 170-172 °C.

### ETAPE A₄

### N-[(5,6-diméthoxy indan-2 yl)méthyl] N-(méthyl)amine.

On ajoute, à une suspension de 1,7 g d'hydrure de lithium et d'aluminium dans 25 cm³ de tétrahydrofurane, 10,5 g du composé obtenu à l'étape A₃ dans 150 cm³ de tétrahydrofurane.
On porte le milieu réactionnel 24 heures au reflux puis on effectue une étape d'hydrolyse par l'ajout de 1,1 cm³ d'eau, suivie par 0,94 cm³ d'hydroxyde de sodium à 20 %, et enfin par 4,2 cm³ d'eau. On filtre et on évapore sous vide le solvant pour obtenir 3,9 g d'une huile qui correspond au composé du titre.
Rendement : 40 %.

### STADE B : 7,8-diméthoxy 3-[3-(iodo)propyl] 1,3-dihydro 2H 3-benzazépine 2-one.

Préparation identique à celle du stade B de l'exemple 1.

### STADES C et D : En procédant comme dans les stades C et D de l'exemple 1, mais en remplaçant au stade C le composé obtenu au stade A de l'exemple 1 par le composé obtenu au stade A du présent exemple, on obtient successivement :

### STADE C : 7,8-diméthoxy 3-{3-{N-[5,6-diméthoxy indan-2 yl)méthyl] N-(méthyl)amino}propyl} 1,3-dihydro 2H 3-benzazépin 2-one.

Rendement : 61 % (huile).

| Caractéristiques spectrales : RMN (CDCl₃) : | | | | | |
|---|---|---|---|---|---|
| 6,75 ppm | 3 singulets | 4H | 6,3-6,1 ppm | 2 doublets | 2H |
| 3,9 ppm | 2 singulets | 12 H | 3,6 ppm | triplet | 2H |
| 3,4 ppm | singulet | 2H | 3,4 ppm | singulet | 2H |
| 3 ppm | multiplet | 2H | 2,7-2,5 ppm | 2 multiplets | 3H |
| 2,25 ppm | 2 multiplets | 4H | 2,15 ppm | singulet | 3 H |
| 1,7 ppm | multiplet | 2H | | | |

### STADE D : 7,8-diméthoxy 3-{3-{N-[5,6-diméthoxy indan-2 yl)méthyl] N-(méthyl)amino}propyl} 1,3,4,5-tétrahydro 2H 3-benzazépin 2-one.

Rendement : 44 %.
Point de fusion : 98-100 °C.
Solvant de recristallisation : acétate d'éthyle.

| Caractéristiques spectrales : RMN (CDCl₃) : | | | | | |
|---|---|---|---|---|---|
| 6,7 ppm | singulet | 2H | 6,55 ppm | singulet | 1H |
| 3,85 ppm | singulet | 12 H | 3,8 ppm | multiplet | 2H |
| 3,75 ppm | multiplet | 2H | 3,5 ppm | triplet | 2H |
| 3,25 ppm | quintuplet | 1H | 3,05 ppm | multiplet | 2H |
| 2,8 ppm | multiplet | 4H | 2,4 ppm | multiplet | 2H |
| 2,25 ppm | singulet | 3H | 1,8 ppm | quintuplet | 2H |

### EXEMPLE 10

### 7,8-diméthoxy 3-{3-{N-[(5,6-diméthoxy indan-2 yl)méthyl]amino}propyl} 1,3,4,5-tétrahydro 2H 3-benzazépin 2-one.

En procédant comme dans l'exemple 9, mais en remplaçant à l'étape A₃ la méthylamine par l'ammoniac, on obtient successivement :

### STADE A : N-[(5,6-diméthoxy indan-2 yl)méthyl]amine.

### ETAPE A₁

5,6-(diméthoxy) 2-(éthoxy carbonyl) indan 1-one.

### ETAPE A₂

Acide (5,6-diméthoxy indan) 2-oïque.

### ETAPE A₃

(5,6-diméthoxy indan-2 yl)carboxamide.
Rendement : 85,4 %.
Point de fusion : 190-192 °C.

### STADE B : 7,8-diméthoxy 3-[3-(iodo)propyl] 1,3-dihydro 2H 3-benzazépine 2-one.

### STADE C : 7,8-diméthoxy 3-{3-{N-[(5,6-diméthoxy indan-2 yl)méthyl]amino} propyl} 1,3-dihydro 2H 3-benzazépin-2 one.

Rendement : 33 % (huile).

### STADE D : 7,8-diméthoxy 3-{3-{N-[(5,6-diméthoxy indan-2 yl)méthyl]amino} propyl} 1,3,4,5-tétrahydro 2H 3-benzazépin 2-one.

Point de fusion : 86-94 °C.
Solvant de recristallisation : acétate d'éthyle.

| Caractéristiques spectrales : RMN (CDCl₃) : | | | | | |
|---|---|---|---|---|---|
| 6,7 ppm | singulet | 2H | 6,6 ppm | singulet | 1H |
| 6,55 ppm | singulet | 1H | 3,8 ppm | singulet | 14 H |
| 3,7 ppm | multiplet | 2H | 3,5 ppm | triplet | 2H |
| 3,05 ppm | multiplet | 2H | 3,0 ppm | multiplet | 2H |
| 2,9-2,5 ppm | multiplet | 7H | 1,8 ppm | qintuplet | 2H |

### EXEMPLE 11

### 7,8-diméthoxy 3-{3-{[(4-méthoxy benzocyclobut-1 yl)méthyl] N-(méthyl)amino} propyl} 1,3,4,5-tétrahydro 2H 3-benzazépin 2-one.

En opérant comme dans l'exemple 1 : à partir de 8,7 g de chlorhydrate de N[(4-méthoxy benzocyclobut-1-yl)méthyl]amine, 4,2 ml de chloroformiate d'éthyle, 12 ml de triéthylamine et 90 ml de dichlorométhane, on obtient 11 g de N-[(4-méthoxybenzocyclobut-1 yl)méthyl] N-(éthoxycarbonyl)amine (Rendement théorique).

A partir de 11 g de ce produit ainsi obtenu, 3,3 g de LiAlH₄ et 160 ml de tétrahydrofurane chauffés à reflux pendant 6 heures, on obtient 7 g de N-[(4-méthoxy benzocyclobut-1 yl)méthyl] N-(méthyl) amine, (Rendement 90%).

A partir de 15 g de 7,8-diméthoxy 3-[3-(iodo)propyl] 1,3-dihydro 2H 3-benzazépin 2-one, 7 g de N-[(4-méthoxy benzocyclobut-1 yl) méthyl] N-(méthyl)amine, 22 g de K₂CO₃ et 300 ml d'acétone, on obtient, avec un rendement théorique, 17,5 g de 7,8-diméthoxy-3-**{**3-{N-[(4-méthoxy benzoycyclobut-1 yl) méthyl] N-(méthyl) amino} propyl**}** 1,3-dihydro 2H-3-benzazépin 2-one.

A partir de 5 g de ce produit ainsi obtenu, 60 ml d'éthanol, 1 ml d'acide acétique et 3 g d'hydroxyde de palladium, on obtient 1,9 g de 7,8-diméthoxy-3-**{**3-{N-[(4-méthoxy benzocyclobut-1 yl) méthyl] N-(méthyl)amino} propyl**}** 1,3,4,5-tétrahydro 2H 3-benzazépin 2-one (Rendement : 65 %), que l'on transforme en dichlorhydrate correspondant qui, recristallisé dans l'acétonitrile, fond à 160-164 °C.

### EXEMPLE 12

### 7,8-diméthoxy-3-{3-{N-[2-(5,6-diméthoxyindan-2 yl) éthyl] N-(méthyl) amino} propyl} 1,3,4,5-tétrahydro 2H 3-benzazépin 2-one.

En opérant comme dans l'exemple 9 :
A partir de 45 g d'acide (5,6-diméthoxy indan)2-oïque et 7,7 g de LiAlH₄, on obtient, après 18 heures à température ambiante puis hydrolyse et concentration, 35 g de (5,6-diméthoxy indan-2 yl) méthanol (rendement : 85 %).

A partir de 10 g de l'alcool ainsi obtenu, 13 g de paratoluène sulfochlorure et 100 ml de pyridine, que l'on maintient 2 heures à 0 °C puis 12 heures à 20 °C puis lave à l'eau et concentre, on obtient 15 g du paratoluène sulfonate correspondant. Ce dernier traité par 6 g de NaCN dans 105 ml de DMSO, pendant 8 heures à 80 °C puis concentration et reprise par eau/ether, permet d'obtenir avec un rendement de 82 %, 9 g de cyanure de (5,6-diméthoxyindan-2-yl) méthyle.

A partir de 25 g de cyanure de 5,6-diméthoxyindan-2 yl) méthyle, chauffés à reflux pendant 18 heures avec 20 g de KOH, 250 ml d'éthanol et 31 ml d'eau, puis concentration du mélange réactionnel, acidification et extraction, on obtient 24 g d'acide (5,6-diméthoxyindan-2 yl) acétique.

A partir de 31 g d'acide (5,6-diméthoxyindan-2 yl) acétique, 400 ml de chlorure de méthylène et 21,3 g de carbonyldiimidazole, agités ensemble pendant 2 heures à température ambiante, puis saturation du mélange réactionnel par de la méthylamine pendant 4 heures et enfin lavage à l'eau et concentration, on obtient 33 g de N-[(5,6-diméthoxy indan-2 yl) méthyl carbonyl] N-(méthyl) amine (Rendement théorique).

37 g de N-[(5,6-diméthoxy indan-2 yl) méthylcarbonyl] N-(méthyl) amine sont traités par 8,5 g de LiAlH₄ dans 470 ml de tétrahydrofurane à reflux pendant 6 heures, puis le mélange est hydrolysé puis concentré pour donner finalement 24 g de N-[(5,6-diméthoxy indan-2-yl) éthyl] N-(méthyl) amine (Rendement 69 %).

A partir de 7 g de l'amine ainsi obtenue, 8,7 g de 7,8-diméthoxy 3-[3-iodopropyl] 1,3-dihydro 2H 3-benzazépin 2-one, 400 ml de cyanure de méthyle et 16,5 g de carbonate de potassium, on obtient 12,5 g de 7,8-diméthoxy 3-**{**3-{N-[(4,5-diméthoxy indan-2 yl éthyl] N-(méthyl) amino} propyl} 1,3-dihydro-2H-3-benzazépin-2-one (Rendement 85 %).
Cette dernière additionnée à 180 ml d'éthanol et 6 ml d'acide acétique est hydrogénée en présence de 6 g d'hydroxyde de palladium pour donner le 7,8-diméthyl-3-{3-{N-[2-(5,6 diméthoxy indan-2 yl) éthyl] N-(méthyl) amino} propyl} 1,3,4,5-tétrahydro 2H 3-benzazépin 2-one, P.F (MK) : 102-107 °C (éther isopropylique) (Rendement : 7 %).

### EXEMPLE 13

### 7,8-diméthoxy 3-{3-{N-[(4,5-diméthoxy 1-méthyl benzocyclobutan-1-yl) méthyl] N-(méthyl) amino} propyl} 1,3,4,5-tétrahydro 2H 3-benzazépin 2-one.

En opérant comme dans l'exemple 9 :
A partir de 20 g de cyanure de 4,5-diméthoxy benzocyclobut-1-yle, 35 ml de diisopropyl amine, 2,5 mole de butyllithium dans 100 ml d'hexane, 170 ml de tétrahydrofurane et 123 ml d'iodure de méthyle, on obtient 22 g de cyanure de 4,5-diméthoxy 1-méthyle benzocyclobutan-1-yle, sous forme d'huile (Rendement 97 %) qui, traité pendant 4 heures à reflux avec 17,5 g de potasse, 220 ml d'éthanol et 27 ml d'eau, puis concentration du mélange, acidification et extraction à l'acétate d'éthyle donnent 20 g d'acide (4,5-diméthoxy 1-méthyl benzocyclobutan) 1-oïque sous forme d'huile (Rendement 92 %).

Ces 20 g d'acide sont agités une nuit avec 14,6 g de carbonyldiimidazole dans 200 ml de chlorure de méthylène, puis l'ensemble est saturé par 15 g de méthylamine. Après lavage à l'eau et concentration, on obtient 9,7 g de N-[(4,5-diméthoxy 1-méthyl benzocyclobutan-1yl) carbonyl] N-méthylamine (Rendement 47,6 %) lesquels traités 24 heures à température ambiante puis 4 heures à reflux par 2,6 g de LiAlH₄ dans 140 ml de tétrahydrofurane donnent, après hydrolyse du mélange réactionnel au moyen d'eau et de soude, puis concentration, 9 g de N-[(4,5-diméthoxy 1-méthyl benzocyclobutan-1 yl) méthyl] N-(méthyl) amine (Rendement 95 %).

A partir de 4,4 g de l'amine ainsi obtenue et 7,7 g de 7,8-diméthoxy 3-[3-(iodo) propyl] 1,3-dihydro 2H 3-benzazépin 2-one dans 160 ml d'acétone en présence de 9,1 g de carbonate de potassium, en opérant comme dans l'exemple 1 stade C, on obtient 12,4 g de 7,8-diméthoxy 3-{3-{N-[(4,5-diméthoxy 1-méthyl benzocyclobutan-1-yl) méthyl] N-(méthyl) amino} propyl**}** 1,3-dihydro 2H 3-benzazépin 2-one (Rendement 95 %).

A partir de 12 g de ce dernier produit, 125 ml d'éthanol, 2,6 ml d'acide acétique et 6 g d'hydroxyde de palladium, on obtient, en opérant comme dans l'exemple 1 stade D, 3,3 g de 7,8-diméthoxy 1-méthyl benzocyclobutan-1-yl) méthyl] N-(méthyl) amino} propyl} 1,3,4,5-térahydro 2H 3-benzazépin 2-one, PF (MK) : 105-108 °C.

### EXEMPLE 14

7,8-diméthoxy 3-{3-{N[(furo[2,3-b]benzocyclobutan 6-yl) méthyl] N-(méthyl) amino} propyl} 1,3,4,5-tétrahydro 2H 3-benzazépin 2-one.

A partir de 25 g de chlorhydrate de [5-méthoxy benzocyclobutan-1-yl) méthyl] amine, traités une nuit sous agitation avec 41,7 ml de triéthylamine et 10,6 ml de chlorure d'acétyle dans 300 ml de chlorure de méthylène, on obtient 25,8 g de N-[(5-méthoxy benzocyclobutan 1-yl) méthyl] N-acétyl amine, huile (Rendement théorique).

25,6 g du produit ainsi obtenu, dans 375 ml de CHCl₃ sont traités sous agitation à 0 °C pendant 2 heures par 130 ml de BBr₃, puis après ajout d'éthanol, concentration et lavage, on obtient 23 g de N-[5-hydroxy benzocyclobutan 1-yl) méthyl] N-acétyl amine, huile (Rendement 98 %).

L'ensemble du produit ainsi obtenu est traité pendant 4 heures à 0 °C avec 13 ml de ClCH₂CN, 297 ml d'une solution molaire de BCl₃ dans CHCl₂ et 16,6 g d'AlCl₃ et 65 ml de CH₂Cl₂.

L'ensemble est ensuite lavé à l'eau puis concentré pour obtenir 21 g de N-[3-oxo 2,3-dihydro furo [2,3-b] benzocyclobutan 6-yl) méthyl] N-acétyl amine, huile (Rendement 75 %).

Ces 21 g de produit sont traités 17 heures à température ambiante par 6,4 g de NaBH₃ dans 210 ml de méthanol et 110 ml de NaHCO₃. Après dilution du mélange avec HCl puis extraction à l'acétate d'éthyle, on obtient 15,8 g de N-[furo[2,3-b] benzocyclobutan 6-yl méthyl] N-acétyl amine, PF (MK) : 100-102 °C (Rendement 75 %).

Ces 15,8 g d'amine sont maintenus pendant 20 heures à reflux avec 700 ml de méthanol, 220 ml d'HCl concentré et 220 ml d'eau. Puis le mélange réactionnel est concentré et repris par CH3CN pour donner 9,3 g de chlorhydrate de N-[(furo[2,3-b] benzocyclobutan 6-yl) méthyl] amine, PF (MK) : 268-270 °C (Rendement 60 %).

2,25 g de ce chlorhydrate sont mis à réagir pendant 21 heures à température ambiante avec 1,13 ml de ClCOOC₂H₅, 3,3 ml de triéthylamine et 3,3 ml de chlorure de méthylène. Après lavage à l'eau et concentration, on obtient 1,65 g de N-[(furo[2,3-b] benzocyclobutan 6-yl) méthyl] N-(éthyloxy carbonyl) amine, huile (Rendement 69 %).

L'ensemble de ce produit est traité 6 heures à reflux avec 0,5 g de LiAlH₄ dans 30 ml de tétrahydrofurane ; puis le mélange réactionnel est hydrolysé puis concentré pour donner 1,3 g de N-(furo[2,3-b] benzocyclobutan 6-yl méthyl) N-méthyl amine (Rendement théorique).

1,2 g de N-[(furo[2,3-b] benzocyclobutan 6-yl) méthyl] N-méthyl amine, 1,9 g de 7,8-diméthoxy 3 {3-chloropropyl} 1,3,4,5-tétrahydro 2H 3-benzazépin 2-one et 13 ml de triéthylamine sont chauffés 3 heures à 60 °C, puis 1 heure à reflux. Après extraction et lavage à la soude du mélange réactionnel, on obtient 1 g de 7,8-diméthoxy 3-{3-{N[(furo[2,3-b] benzocyclobutan 6-yl) méthyl] N-(méthyl) amino} propyl} 1,3,4,5-tétrahydro 2H 3-benzazépin 2-one (Rendement 31 %), PF (MK) du dichlorhydrate correspondant : 180-188 °C.

### EXEMPLE 15

### ETUDE PHARMACOLUGIQUE

### A - ETUDE IN VIVO

### Effets hémodynamiques des composés de l' invention chez le rat vigile

### Protocole d'étude

Des rats Wistar mâles (350-400 g) sont anesthésiés par administration en intrapéritonéal d'un mélange de kétamine (Imalgène® 1000 ; 140 mg/kg) et d'acépromazine (Vératranquil® 1 % ; 14 mg/kg). L'artère fémorale et la veine jugulaire sont cathétérisées, respectivement pour la mesure de la pression artérielle et de la fréquence cardiaque et pour l'injection intraveineuse des composés testés. Les animaux sont utilisés après une période post-opératoire de 48 heures.
Le jour de l'expérience, une période de stabilisation d'une heure des paramètres hémodynamiques est respectée. Les traitements sont administrés par voie intraveineuse ou par voie orale. Un groupe témoin reçoit le solvant utilisé, dans les mêmes conditions expérimentales.
La fréquence cardiaque et la pression artérielle moyenne sont suivies en continu jusqu'à 6 heures après traitement.

### Résultats

### Effet des composés de l'invention, administrés par voie intraveineuse, sur la fréquence cardiaque (EC) de rats vigiles

| | **Doses (mg/kg)** | **Evolution FC (Δ %)** | | | |
|---|---|---|---|---|---|
| | | 30 min | 1 h | 4 h | 6 h |
| **Contrôle** | | 1 ± 2 | 2 ± 2 | -3 ± 1 | -5 ± 1 |
| **Exemple 1** | 0,5 | -9 ± 4 | -10 ± 2 | -12 ± 4 | -17 ± 3 |
| | 1 | -18 ± 3 | -21 ± 3 | -16 ± 5 | -16 ± 3 |
| | 2 | -29 ± 2 | -26 ± 3 | -14 ± 2 | -16 ± 1 |
| **Exemple 9** | 0,5 | -14 ± 4 | -16 ± 5 | -12 ± 3 | -14 ± 2 |
| | 1 | -31 ± 6 | -32 ± 7 | -21 ± 5 | -21 ± 4 |
| | 2 | -34 ± 3 | -35 ± 4 | -24 ± 4 | -24 ± 4 |

### Effet des composés de l'invention, administrés par voie orale, sur la fréquence cardiaque (FC) de rats vigiles

| | **Doses (mg/kg)** | **Evolution FC (Δ %)** | | |
|---|---|---|---|---|
| | | 1 h | 3 h | 6 h |
| **Contrôle** | | -4 ± 1 | -3 ± 1 | -8 ± 1 |
| **Exemple 1** | 1,5 | -14 ± 4 | -19 ± 4 | -19 ± 2 |
| | 3 | -22 ± 4 | -28 ± 3 | -26 ± 1 |
| | 6 | -14 ± 3 | -26 ± 3 | -29 ± 4 |
| **Exemple 9** | 1,5 | -10 ± 5 | -13 ± 5 | -19 ± 4 |
| | 3 | -15 ± 2 | -31 ± 3 | -35 ± 3 |
| | 6 | -16 ± 3 | -27 ± 5 | -37 ± 3 |

Les composés de l'invention ont une activité bradycardisante puissante et de longue durée tant après administration par voie intraveineuse que par voie orale.

Cet effet sur la fréquence cardiaque ne s'accompagne pas d'effet déletère sur la pression artérielle.

### B - ETUDE IN VITRO

### Effet sur la fréquence- spontanée d'oreillette droite de rat

### Protocole d'étude

Les coeurs de rats Wistar mâles (325-350 g) anesthésiés au pentobarbital sodique (30 mg/kg IP) sont rapidement prélevés, les oreillettes droites sont isolées et accrochées à un capteur de tension Statham® (UC₂-Gould) avec une tension initiale de 0,5 g. La fréquence de l'activité contractile spontanée est calculée par l'intermédiaire d'un compteur Biotach-Gould.

La solution physiologique utilisée a la composition suivante (mM) NaCl 112 ; KCl 5 ; KH₂PO₄ 1 ; MgSO₄ 1,2 ; CaCl₂ 2,5 ; NaHCO₃ 25 ; Glucose 11,5 ; EDTA 0,026 ; pH 7,4. Elle est aérée par un mélange de 95 % 02 - 5 % CO₂ et thermostatée à 35 °C.

Après 30 minutes de stabilisation, des concentrations cumulatives des composés testés sont additionnées au milieu toutes les 30 minutes.

### Résultats

Les composés de l'invention réduisent de façon puissante,concentrationdépendante, la fréquence spontanée des oreillettes droites isolées.

A titre d'exemple, les composés des exemples 1 et 9 réduisent de 43 % et de 68 % la fréquence des oreillettes à la concentration de 3.10⁻⁶ M.

Ces expériences démontrent que l'activité bradycardisante des composés de l'invention résulte d'une activité directe sur le noeud sinusal responsable de l'activité pacemaker cardiaque.

## Revendications (Revendications pour l'(les) Etat(s) contractant(s) suivant(s): AT, BE, CH, DE, DK, FR, GB, IT, LI, LU, NL, SE)

1. Les composés de formule générale I : 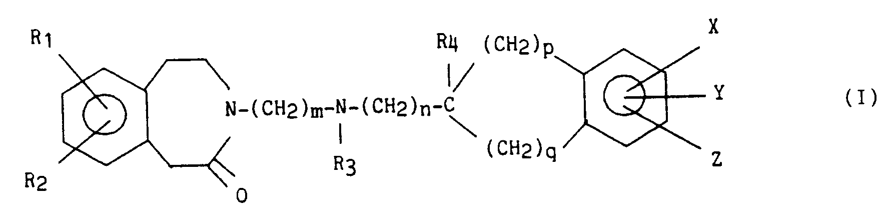 dans laquelle :
m représente un nombre entier de 2 à 5,
n représente un nombre entier de 1 à 6,
p et q, identiques ou différents, représentent 0 ou un nombre entier de 1 à 2,
à condition que la somme p + q soit égale à 1 ou 2,
R₁ et R₂, identiques ou différents, représentent un groupement choisi parmi :
- hydrogène,
- halogène,
- hydroxy,
- alcoxy inférieur,
- phénylalcoxy inférieur,
- et phénylalcoxy inférieur substitué,
ou R₁ et R₂ forment ensemble, lorsqu'ils sont portés par 2 atomes de carbone adjacents, un groupement -O-(CH₂)ᵣ-O- avec r représentant un nombre entier égal à 1 ou 2,
R₃ représente un groupement choisi parmi :
- hydrogène,
- alkyl inférieur,
- alkényl inférieur,
- cycloalkyle,
- cycloalkyalkyle inférieur,
- phénylalkyle inférieur,
- phénylalkyle inférieur substitué,
- -CO-R₅ ou -CO-O-R₅ avec R₅ signifiant un groupement choisi parmi : hydrogène, alkyle inférieur, alkényle inférieur, alkinyle inférieur, phényle, phényle substitué, phénylalkyle inférieur, phénylalkyle inférieur substitué, cycloalkyle inférieur, et cycloalkylalkyle inférieur,
- et -CO-NR₆R₇, avec R₆ et R₇, identiques ou différents, ayant les mêmes significations que celles du groupement R₅ tel que défini ci-dessus,
ou R₆ et R₇ forment ensemble, avec l'atome d'azote qui les porte, un cycle saturé de 4 à 7 chainons,
R₄ représente un atome d'hydrogène ou un radical alkyle inférieur,
X, Y, Z, identiques ou différents, représentent un groupement choisi parmi :
- hydrogène,
- halogène,
- hydroxy,
- alcoxy inférieur,
- phénylalcoxy inférieur substitué,
ou X et Y, ou Y et Z, forment ensemble, lorsqu'ils sont portés par 2 carbones adjacents, un groupement -O-(CH₂)ᵣ-O- dans lequel r représente un nombre entier égal à 1 ou 2, un groupement -O-(CH₂)₂- ou un groupement -O-CH=CH- ;
étant entendu que le terme "substitué" affectant les groupements "phényle", "phénylalkyle inférieur" et "phénylalcoxy inférieur" signifie que ces groupements peuvent être substitués, sur le noyau phényle, par un ou plusieurs substituants choisis parmi : les atomes d'halogène, et les radicaux : hydroxy, trifluorométhyle, alkyle inférieur, et alcoxy inférieur,
étant entendu - que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkinyle inférieur" désignent des groupements insaturés linéaires ou ramifiés contenant de 2 à 6 atomes de carbone,
- que le terme "cycloalkyle" désigne un cycle hydrocarboné saturé contenant de 3 à 8 chainons,
leurs éventuels isomères optiques, isolés ou sous forme de mélange, ainsi que, le cas échéant, leurs sels d'addition à un acide pharmaceutiquement acceptable.

2. Le composé selon la revendication 1 qui est le (R,S) 7,8-diméthoxy 3-{3-{N-[(4,5-diméthoxy benzocyclobut-1 yl)méthyl] N-(méthyl)amino}propyl} 1,3,4,5-tétahydro 2H 3-benzazépin 2-one,
ses isomères optiques, isolés ou sous forme de mélange, et ses sels d'addition à un acide pharmaceutiquement acceptable.

3. Le composé selon la revendication 1 qui est le (R,S) 7,8-diméthoxy 3-{3-{N-[(5-méthoxy benzocyclobut-1 yl)méthyl] N-(méthyl)amino}propyl} 1,3,4,5-tétrahydro 2H 3-benzazépin 2-one, ses isomères optiques,isolés ou sous forme de mélange, et ses sels d'addition à un acide pharmaceutiquement acceptable.

4. Le composé selon la revendication 1 qui est le (R,S) 7,8-diméthoxy 3-{3-{N-[(benzocyclobut-1 yl)méthyl] N-(méthyl)amino}propyl} 1,3,4,5-tétrahydro 2H 3-benzazépin 2-one, ses isomères optiques, isolés ou sous forme de mélange, et ses sels d'addition à un acide pharmaceutiquement acceptable.

5. Le composé selon la revendication 1 qui est le 7,8-diméthoxy 3-{3-{N-[(5,6-diméthoxy indan-2 yl)méthyl] N-(méthyl)amino}propyl} 1,3,4,5-tétrahydro 2H 3-benzazépin 2-one,
et ses sels d'addition à un acide pharmaceutiquement acceptable.

6. Le composé selon la revendication 1 qui est le 7,8-diméthoxy 3-{3-{N-[5,6-diméthoxy indan-2 yl)méthyl]amino}propyl} 1,3,4,5-tétrahydro 2H 3-benzazépin 2-one,
et ses sels d'addition avec un acide pharmaceutiquement acceptable.

7. Le procédé de préparation des composés de formule (I), caractérisé en ce que :
on condense une amine de formule (II) : 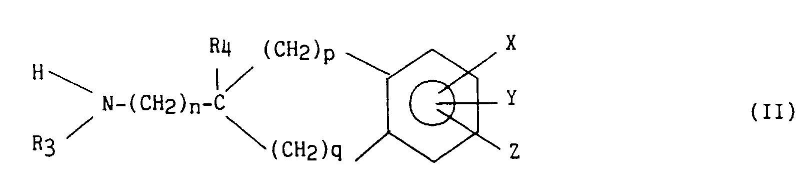 dans laquelle R₃, R₄, X, Y, Z, n,p et q sont tels que définis dans la formule (I) selon la revendication 1, sous forme racémique ou optiquement active lorsqu'il existe un carbone asymétrique,
avec un composé de formule (III) : 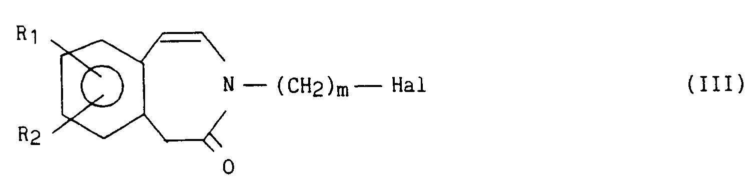 dans laquelle R₁, R₂, et m sont tels que définis dans la formule (I) selon la revendication 1, et Hal représente un atome d'halogène,
pour obtenir un composé de formule (IV) : 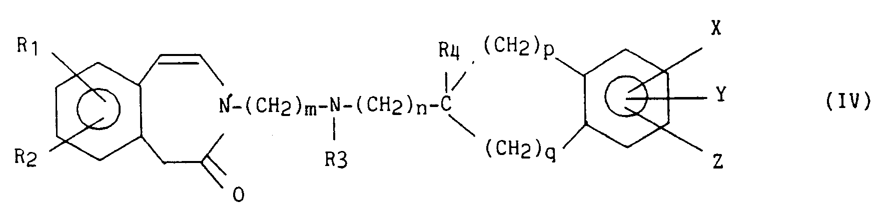 dans laquelle R₁, R₂, R₃, R₄, X, Y, Z, m, n, p et q sont tels que définis précédemment, sous forme racémique ou optiquement active lorsqu'il existe un carbone asymétrique,
composé de formule IV qui est soumis à une hydrogénation afin d'obtenir un composé de formule (I) : 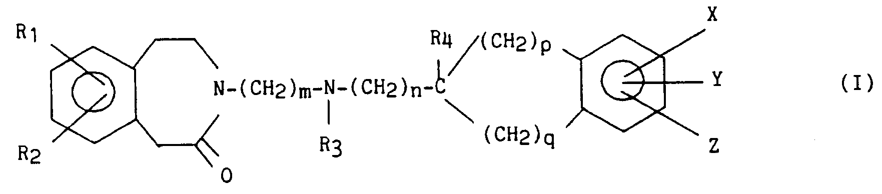 dans laquelle R₁, R₂, R₃, R₄, X, Y, Z, m, n, p et q sont tels que définis précédemment, sous forme racémique ou optiquement active lorsqu'il existe un carbone asymétrique,
lequel composé de formule (I) est, si on le désire, :
- purifié par une technique classique de cristallisation et/ou de chromatographie,
- et/ou salifié avec un acide pharmaceutiquement acceptable,
étant entendu que les composés de formule (I), lorsqu'ils possèdent un carbone asymétrique peuvent être préparés sous une forme optiquement active non seulement en utilisant des matières premières optiquement actives mais également à partir des composés racémiques de formule (I) correspondants, par des méthodes classiques de séparation des isomères optiques.

8. Composé de formule (IV) : 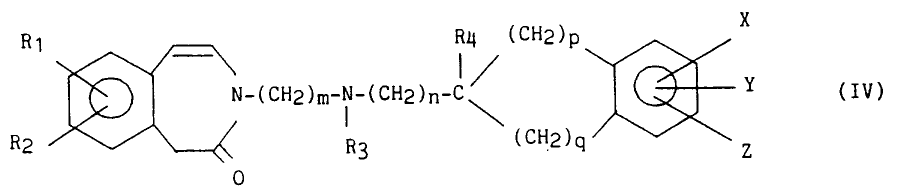 dans laquelle R₁, R₂, R₃, R₄, X, Y, Z, m, n, p et q sont tels que définis dans la formule (I) selon la revendication 1,
utiles comme matières premières dans la synthèse des composés de l'invention de formule (I) selon la revendication 1, leurs éventuels isomères optiques, isolés ou sous forme de mélange, et leurs sels d'addition à un acide.

9. Composition pharmaceutique contenant comme principe actif au moins un composé selon l'une des revendications 1 à 6 en combinaison avec un ou plusieurs excipients ou véhicules inertes, non toxiques, et pharmaceutiquement acceptables.

10. Composition pharmaceutique selon la revendication 9, utilisable dans la prévention et le traitement des différentes expressions cliniques de l'ischémie myocardique qui résultent d'un déséquilibre entre l'apport et la demande en oxygène myocardique telles que l'angine de poitrine, l'infarctus du myocarde et les troubles du rythme associés, ainsi que dans la prévention et le traitement des pathologies comportant des troubles du rythme, notamment supra-ventriculaires, et dans les complications des lésions athéroscléreuses notamment coronaires par limitation des contraintes hémodynamiques vasculaires.

## Revendications (Revendications pour l'(les) Etat(s) contractant(s) suivant(s): ES, GR)

1. Le procédé de préparation des composés de formule générale I : 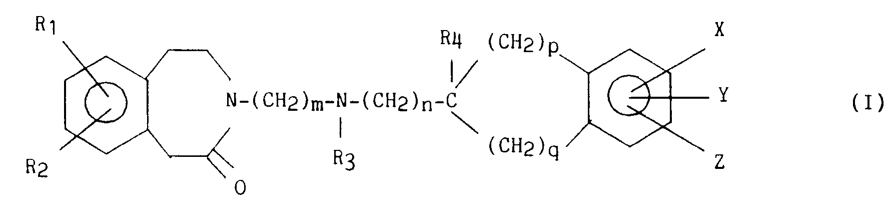 dans laquelle :
m représente un nombre entier de 2 à 5,
n représente un nombre entier de 1 à 6,
p et q, identiques ou différents, représentent 0 ou un nombre entier de 1 à 2,
à condition que la somme p + q soit égale à 1 ou 2,
R₁ et R₂, identiques ou différents, représentent un groupement choisi parmi :
- hydrogène,
- halogène,
- hydroxy,
- alcoxy inférieur,
- phénylalcoxy inférieur,
- et phénylalcoxy inférieur substitué,
ou R₁ et R₂ forment ensemble, lorsqu'ils sont portés par 2 atomes de carbone adjacents, un groupement -O-(CH₂)ᵣ-O- avec r représentant un nombre entier égal à 1 ou 2,
R₃ représente un groupement choisi parmi :
- hydrogène,
- alkyl inférieur,
- alkényl inférieur,
- cycloalkyle,
- cycloalkyalkyle inférieur,
- phénylalkyle inférieur,
- phénylalkyle inférieur substitué,
- -CO-R₅ ou -CO-O-R₅ avec R₅ signifiant un groupement choisi parmi : hydrogène, alkyle inférieur, alkényle inférieur, alkinyle inférieur, phényle, phényle substitué, phénylalkyle inférieur, phényialkyle inférieur substitué, cycloalkyle inférieur, et cycloalkylalkyle inférieur,
- et -CO-NR₆R₇, avec R₆ et R₇, identiques ou différents, ayant les mêmes significations que celles du groupement R₅ tel que défini ci-dessus,
ou R₆ et R₇ forment ensemble, avec l'atome d'azote qui les porte, un cycle saturé de 4 à 7 chainons,
R₄ représente un atome d'hydrogène ou un radical alkyle inférieur,
X, Y, Z, identiques ou différents, représentent un groupement choisi parmi :
- hydrogène,
- halogène,
- hydroxy,
- alcoxy inférieur,
- phénylalcoxy inférieur substitué,
ou X et Y, ou Y et Z, forment ensemble, lorsqu'ils sont portés par 2 carbones adjacents, un groupement -O-(CH₂)ᵣ-O- dans lequel r représente un nombre entier égal à 1 ou 2, un groupement -O-(CH₂)₂- ou un groupement -O-CH=CH- ;
étant entendu que le terme "substitué" affectant les groupements "phényle", "phénylalkyle inférieur" et "phénylalcoxy inférieur" signifie que ces groupements peuvent être substitués, sur le noyau phényle, par un ou plusieurs substituants choisis parmi : les atomes d'halogène, et les radicaux : hydroxy, trifluorométhyle, alkyle inférieur, et alcoxy inférieur,
étant entendu - que les termes "alkyle inférieur" et "alcoxy inférieur" signifient des groupements carbonés saturés linéaires ou ramifiés contenant de 1 à 6 atomes de carbone,
- que les termes "alkényle inférieur" et "alkinyle inférieur" désignent des groupements insaturés linéaires ou ramifiés contenant de 2 à 6 atomes de carbone,
- que le terme "cycloalkyle" désigne un cycle hydrocarboné saturé contenant de 3 à 8 chainons,
leurs éventuels isomères optiques, ainsi que de leurs sels d'addition à un acide pharmaceutiquement acceptable,
caractérisé en ce que :
on condense une amine de formule (II) : 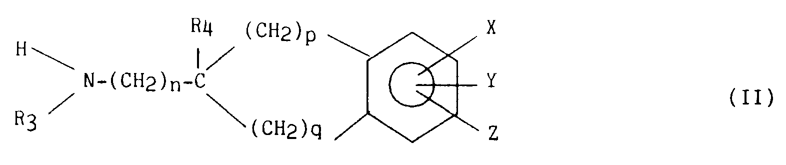 dans laquelle R₃, R₄, X, Y, Z, n,p et q sont tels que précédemment définis, sous forme racémique ou optiquement active lorsqu'il existe un carbone asymétrique,
avec un composé de formule (III) : 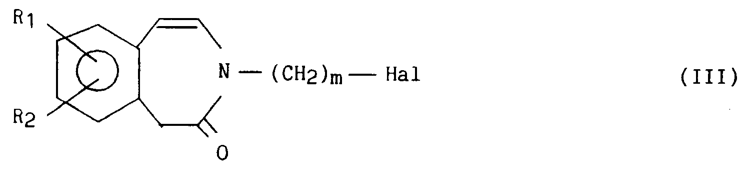 dans laquelle R₁, R₂, et m sont tels que précédemment définis, et Hal représente un atome d'halogène,
pour obtenir un composé de formule (IV) : 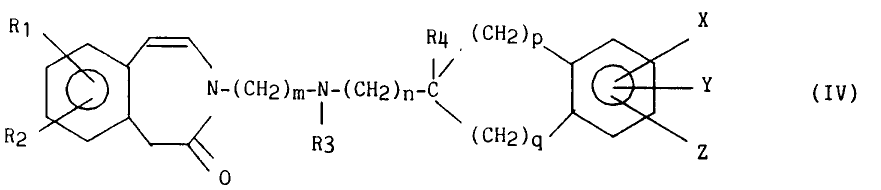 dans laquelle R₁, R₂, R₃, R₄, X, Y, Z, m, n, p et q sont tels que définis précédemment, sous forme racémique ou optiquement active lorsqu'il existe un carbone asymétrique,
et l'on soumet le composé de formule IV ainsi obtenu à une hydrogénation afin d'obtenir un composé de formule (I),
lequel composé de formule (I) est, si on le désire, :
- purifié par une technique classique de cristallisation et/ou de chromatographie,
- et/ou salifié avec un acide pharmaceutiquement acceptable,
étant entendu que les composés de formule (I), lorsqu'ils possèdent un carbone asymétrique peuvent être préparés sous une forme optiquement active non seulement en utilisant des matières premières optiquement actives mais également à partir des composés racémiques de formule (I) correspondants, par des méthodes classiques de séparation des isomères optiques.

## Claims (Claims for the following Contracting State(s): AT, BE, CH, DE, DK, FR, GB, IT, LI, LU, NL, SE)

1. Compounds of the general formula I 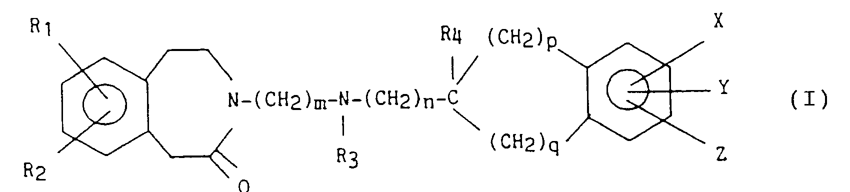 in which
m represents an integer from 2 to 5,
n represents an integer from 1 to 6,
p and g, which are the same or different, each represents 0 or an integer 1 or 2,
with the proviso that the sum of p and q is equal to 1 or 2,
R₁ and R₂, which are the same or different, each represents a group selected from:
- hydrogen,
- halogen,
- hydroxy,
- lower alkoxy,
- phenyl-lower alkoxy,
- and substituted phenyl-lower alkoxy,
or R₁ and R₂, when they are carried by 2 adjacent carbon atoms, together form an -O-(CH₂)ᵣ-O- group in which r represents an integer 1 or 2,
R₃ represents a group selected from:
- hydrogen,
- lower alkyl,
- lower alkenyl,
- cycloalkyl,
- cycloalkyl-lower alkyl,
- phenyl-lower alkyl,
- substituted phenyl-lower alkyl,
- -CO-R₅ or -CO-O-R₅
R₅ representing a group selected from: hydrogen, lower alkyl, lower alkenyl, lower alkynyl, phenyl, substituted phenyl, phenyl-lower alkyl, substituted phenyl-lower alkyl, cyclo-lower alkyl, and cycloalkyl-lower alkyl,
- and -CO-NR₆R₇ wherein R₆ and R₇ are the same or different and each has the same meanings as those of the group R₅ as defined above,
or R₆ and R₇, with the nitrogen atom carrying them, together form a saturated ring having from 4 to 7 chain members,
R₄ represents a hydrogen atom or a lower alkyl radical;
X, Y and Z, which are the same or different, represent a group selected from:
- hydrogen,
- halogen,
- hydroxy,
- lower alkoxy,
- and substituted phenyl-lower alkoxy,
or X and Y, or Y and Z, when they are carried by 2 adjacent carbon atoms, together form an -O-(CH₂)ᵣ-O group in which r represents an integer 1 or 2, an -O-(CH₂)₂- group or an -O-CH=CH- group;
the term "substituted" describing the groups "phenyl", "phenyl-lower alkyl" and "phenyl-lower alkoxy" indicating that those groups may be substituted in the phenyl nucleus by one or more substituents selected from halogen atoms and the radicals hydroxy, trifluoromethyl, lower alkyl and lower alkoxy,
- the terms "lower alkyl" and "lower alkoxy" indicating saturated linear or branched groups containing from 1 to 6 carbon atoms,
- the terms "lower alkenyl" and "lower alkynyl" indicating unsaturated linear or branched groups containing from 2 to 6 carbon atoms,
- the term "cycloalkyl" indicating a saturated hydrocarbon ring containing from 3 to 8 chain members,
their possible optical isomers, isolated or in the form of a mixture, as well as, where applicable, their addition salts with a pharmaceutically acceptable acid.

2. A compound according to claim 1, which is (R,S)-7,8-dimethoxy-3-{3-{N-[(4,5-dimethoxybenzocyclobut-1-yl)methyl]-N-(methyl)amino}propyl}-1,3,4,5-tetrahydro-2H-3-benzazepin-2-one,
its optical isomers, isolated or in the form of a mixture, and its addition salts with a pharmaceutically acceptable acid.

3. A compound according to claim 1, which is (R,S)-7,8-dimethoxy-3-{3-{N-[(5-methoxybenzocyclobut-1-yl)methyl]-N-(methyl)amino}propyl}-1,3,4,5-tetrahydro-2H-3-benzazepin-2-one, its optical isomers, isolated or in the form of a mixture, and its addition salts with a pharmaceutically acceptable acid.

4. A compound according to claim 1, which is (R,S)-7,8-dimethoxy-3-{3-{N-[(benzocyclobut-1-yl)methyl]-N-(methyl)amino}propyl}-1,3,4,5-tetrahydro-2H-3-benzazepin-2-one, its optical isomers, isolated or in the form of a mixture, and its addition salts with a pharmaceutically acceptable acid.

5. A compound according to claim 1, which is 7,8-dimethoxy-3-{3-{N-[(5,6-dimethoxyindan-2-yl)methyl]-N-(methyl)amino}propyl}-1,3,4,5-tetrahydro-2H-3-benzazepin-2-one, and its addition salts with a pharmaceutically acceptable acid.

6. A compound according to claim 1, which is 7,8-dimethoxy-3-{3-{N-[(5,6-dimethoxyindan-2-yl)methyl]amino}propyl}-1,3,4,5-tetrahydro-2H-3-benzazepin-2-one, and its addition salts with a pharmaceutically acceptable acid.

7. A process for the preparation of the compounds of formula I, characterised in that :
an amine of formula (II): 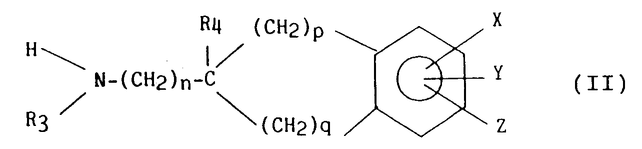 in which R₃, R₄, X, Y, Z, n, p and q are as defined for formula (I) according to claim 1, in racemic or optically active form when there is an asymmetric carbon atom present, is condensed
with a compound of formula (III): 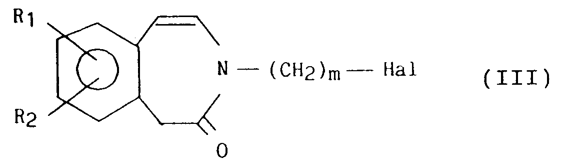 in which R₁, R₂ and m are as defined for formula (I) according to claim 1 and Hal represents a halogen atom,
to yield a compound of formula (IV): 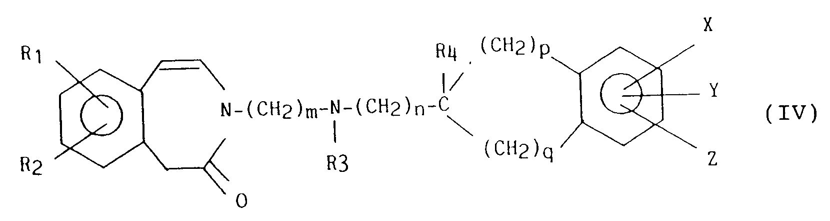 in which R₁, R₂, R₃, R₄, X, Y, Z, m, n, p and q are as defined hereinbefore, in racemic or optically active form when an asymmetric carbon atom is present,
which compound of formula IV is subjected to hydrogenation to yield a compound of formula (I): 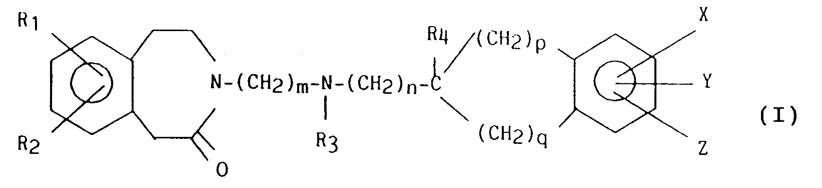 in which R₁, R₂, R₃, R₄, X, Y, Z, m, n, p and q are as defined hereinbefore, in racemic or optically active form when there is an asymmetric carbon atom present,
which compound of formula (I) is, if desired:
- purified by a conventional method of crystallisation and/or chromatography,
- and/or converted into a salt with a pharmaceutically acceptable acid,
it being understood that the compounds of formula (I), when they contain an asymmetric carbon atom, may be prepared in an optically active form, not only by using optically active starting materials but also starting from corresponding racemic compounds of formula (I), by using conventional methods of separation of optical isomers.

8. A compound of formula (IV) : 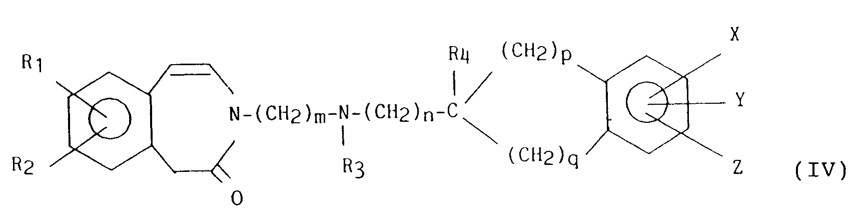 in which R₁, R₂, R₃, R₄, X, Y, Z, m, n, p and q are as defined for formula (I) according to claim 1,
for use as starting materials in the synthesis of compounds of the invention of formula (I) according to claim 1, their possible optical isomers, isolated or in the form of a mixture, and their addition salts with an acid.

9. A pharmaceutical composition comprising as active ingredient at least one compound according to any one of claims 1 to 6 in combination with one or more inert, non-toxic, pharmaceutically acceptable excipients or carriers.

10. A pharmaceutical composition according to claim 9, which can be used in the prevention and treatment of various clinical manifestations of myocardial ischaemia resulting from an imbalance between the supply and demand of myocardial oxygen, such as angina of the chest, myocardial infarction and associated rhythm disorders, as well as in the prevention and treatment of pathologies involving rhythm disorders, especially supra-ventricular rhythm disorders, and in complications of atherosclerotic, especially coronary, lesions by limiting vascular haemodynamic constraints.

## Claims (Claims for the following Contracting State(s): ES, GR)

1. A process for the preparation of compounds of the general formula I 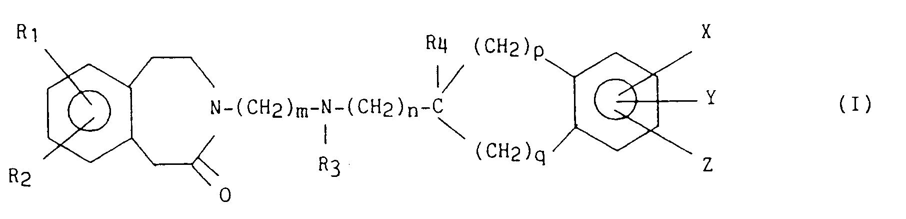 in which
m represents an integer from 2 to 5,
n represents an integer from 1 to 6,
p and q, which are the same or different, each represents 0 or an integer 1 or 2,
with the proviso that the sum of p and q is equal to 1 or 2,
R₁ and R₂, which are the same or different, each represents a group selected from:
- hydrogen,
- halogen,
- hydroxy,
- lower alkoxy,
- phenyl-lower alkoxy,
- and substituted phenyl-lower alkoxy,
or R₁ and R₂, when they are carried by 2 adjacent carbon atoms, together form an -O-(CH₂)ᵣ-O- group in which r represents an integer 1 or 2,
R₃ represents a group selected from:
- hydrogen,
- lower alkyl,
- lower alkenyl,
- cycloalkyl,
- cycloalkyl-lower alkyl,
- phenyl-lower alkyl,
- substituted phenyl-lower alkyl,
- -CO-R₅ or -CO-O-R₅
R₅ representing a group selected from: hydrogen, lower alkyl, lower alkenyl, lower alkynyl, phenyl, substituted phenyl, phenyl-lower alkyl, substituted phenyl-lower alkyl, cyclo-lower alkyl, and cycloalkyl-lower alkyl,
- and -CO-NR₆R₇ wherein R₆ and R₇ are the same or different and each has the same meanings as those of the group R₅ as defined above,
or R₆ and R₇, with the nitrogen atom carrying them, together form a saturated ring having from 4 to 7 chain members,
R₄ represents a hydrogen atom or a lower alkyl radical;
X, Y and Z, which are the same or different, represent a group selected from:
- hydrogen,
- halogen,
- hydroxy,
- lower alkoxy,
- and substituted phenyl-lower alkoxy,
or X and Y, or Y and Z, when they are carried by 2 adjacent carbon atoms, together form an -O-(CH₂)ᵣ-O group in which r represents an integer 1 or 2, an -O-(CH₂)₂- group or an -O-CH=CH- group;
the term "substituted" describing the groups "phenyl", "phenyl-lower alkyl" and "phenyl-lower alkoxy" indicating that those groups may be substituted in the phenyl nucleus by one or more substituents selected from halogen atoms and the radicals hydroxy, trifluoromethyl, lower alkyl and lower alkoxy,
- the terms "lower alkyl" and "lower alkoxy" indicating saturated linear or branched groups containing from 1 to 6 carbon atoms,
- the terms "lower alkenyl" and "lower alkynyl" indicating unsaturated linear or branched groups containing from 2 to 6 carbon atoms,
- the term "cycloalkyl" indicating a saturated hydrocarbon ring containing from 3 to 8 chain members,
their possible optical isomers, and also their addition salts with a pharmaceutically acceptable acid.
characterised in that :
an amine of formula (II): 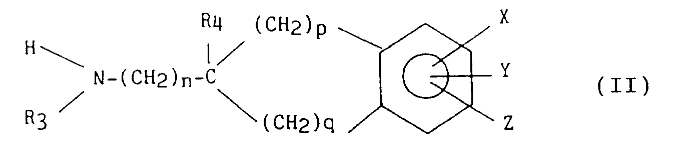 in which R₃, R₄, X, Y, Z, n, p and q are as defined above, in racemic or optically active form when there is an asymmetric carbon atom present, is condensed with a compound of formula (III): 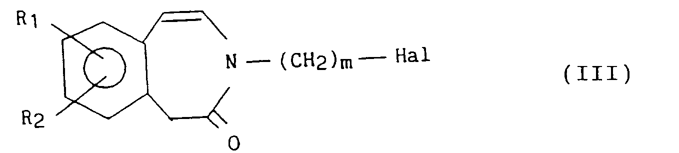 in which R₁, R₂ and m are as defined above, and Hal represents a halogen atom,
to yield a compound of formula (IV): 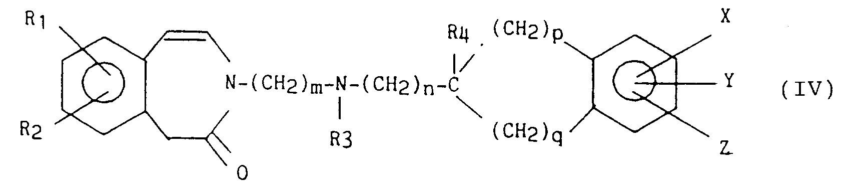 in which R₁, R₂, R₃, R₄, X, Y, Z, m, n, p and q are as defined above, in racemic or optically active form when an asymmetric carbon atom is present,
which compound of formula IV is subjected to hydrogenation to yield a compound of formula (I), which compound of formula (I) is, if desired:
- purified by a conventional method of crystallisation and/or chromatography,
- and/or converted into a salt with a pharmaceutically acceptable acid,
it being understood that the compounds of formula (I), when they contain an asymmetric carbon atom, may be prepared in an optically active form, not only by using optically active starting materials but also starting from corresponding racemic compounds of formula (I), by using conventional methods of separation of optical isomers.

## Patentansprüche (Patentansprüche für folgende(n) Vertragsstaat(en): AT, BE, CH, DE, DK, FR, GB, IT, LI, LU, NL, SE)

1. Verbindungen der allgemeinen Formel I: 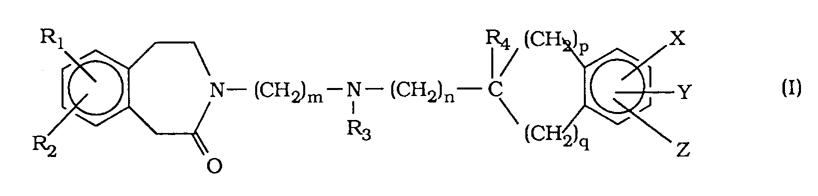 in der:
m eine ganze Zahl mit einem Wert von 2 bis 5,
n eine ganze Zahl mit einem Wert von 1 bis 6,
p und q, die gleichartig oder verschieden sein können, 0 oder eine ganze Zahl mit einem Wert von 1 bis 2,
mit der Maßgabe, daß die Summe von p + q 1 oder 2 beträgt,
R₁ und R₂, die gleichartig oder verschieden sein können, eine Gruppe ausgewählt aus:
- Wasserstoff,
- Halogen,
- Hydroxy,
- Niedrigalkoxy,
- Phenylniedrigalkoxy und
- substituiertes Phenylniedrigalkoxy,
oder R₁ und R₂ gemeinsam, wenn sie von zwei benachbarten Kohlenstoffatomen getragen werden, eine Gruppe -O-(CH₂)ᵣ-O-,
worin r eine ganze Zahl mit einem Wert von 1 oder 2 darstellt,
R₃ eine Gruppe ausgewählt aus:
- Wasserstoff,
- Niedrigalkyl,
- Niedrigalkenyl,
- Cycloalkyl,
- Cycloalkylniedrigalkyl,
- Phenylniedrigalkyl,
- substituiertes Phenylnledrigalkyl,
- -CO-R₅ oder -CO-O-R₅,
worin R₅ eine Gruppe ausgewählt aus: Wasserstoff, Niedrigalkyl, Niedrigalkenyl, Niedrigalkinyl, Phenyl, substituiertes Phenyl, Phenylniedrigalkyl, substituiertes Phenylniedrigalkyl, Niedrigcycloalkyl und Cycloalkylniedrigalkyl darstellt, und
- -CO-NR₆R₇, worin R₆ und R₇, die gleichartig oder verschieden sein können, die gleichen Bedeutungen besitzen wie die oben definierte Gruppe R₅, oder
R₆ und R₇ gemeinsam mit dem Stickstoffatom, das sie trägt, einen gesättigten Ring mit 4 bis 7 Kettengliedern,
R₄ ein Wasserstoffatom oder eine Niedrigalkylgruppe und
X, Y und Z, die gleichartig oder verschieden sein können, eine Gruppe ausgewählt aus:
- Wasserstoff,
- Halogen,
- Hydroxy,
- Niedrigalkoxy,
- substituiertes Phenylniedrigalkoxy, oder
X und Y oder Y und Z gemeinsam, wenn sie von zwei benachbarten Kohlenstoffatomen getragen werden, eine Gruppe -O-(CH₂)ᵣ-O-, in der r eine ganze Zahl mit einem Wert von 1 oder 2 darstellt, eine Gruppe -O-(CH₂)₂- oder eine Gruppe -O-CH=CH- bedeuten;
wobei es sich versteht, daß der Begriff "substituiert" bezüglich der Gruppen "Phenyl", "Phenylniedrigalkyl" und "Phenylniedrigalkoxy" bedeutet, daß diese Gruppen am Phenylkern durch einen oder mehrere Substituenten ausgewählt aus Halogenatomen und Hydroxylgruppen, Trifluormethylgruppen, Niedrigalkylgruppen und Niedrigalkoxygruppen substituiert sein können,
wobei es sich weiterhin versteht,
- daß die Begriffe "Niedrigalkyl" und "Niedrigalkoxy" für geradkettige oder verzweigte gesättigte Kohlenwasserstoffgruppen, die 1 bis 6 Kohlenstoffatome aufweisen, stehen,
- die Begriffe "Niedrigalkenyl" und "Niedrigalkinyl" für geradkettige oder verzweigte ungesättigte Gruppen, die 2 bis 6 Kohlenstoffatome aufweisen, stehen und
- der Begriff "Cycloalkyl" für einen gesättigten Kohlenwasserstoffring mit 3 bis 8 Kettengliedern steht,
deren mögliche optische Isomere in isolierter Form oder in Form einer Mischung, sowie gegebenenfalls ihre Additionssalze mit einer pharmazeutisch annehmbaren Säure.

2. Verbindung nach Anspruch 1, nämlich (R,S)-7,8-Dimethoxy-3-{3-{N-[(4,5-dimethoxy-benzocyclobut-1-yl)-methyl]-N-(methyl)-amino}-propyl}-1,3,4,5-tetrahydro-2H-3-benzazepin-2-on, dessen optische Isomeren in isolierter Form oder in Form einer Mischung und dessen Additionssalze mit einer pharmazeutisch annehmbaren Säure.

3. Verbindung nach Anspruch 1, nämlich (R,S)-7,8-Dimethoxy-3-{3-{N-[(5-methoxy-benzocyclobut-1-yl)-methyl]-N-(methyl)-amino}-propyl}-1,3,4,5-tetrahydro-2H-3-benzazepin-2-on, dessen optische Isomeren in isolierter Form oder in Form einer Mischung und dessen Additionssalze mit einer pharmazeutisch annehmbaren Säure.

4. Verbindung nach Anspruch 1, nämlich (R,S)-7,8-Dimethoxy-3-{3-{N-[(benzocyclobut-1-yl)-methyl]-N-(methyl)-amino}-propyl}-1,3,4,5-tetrahydro-2H-3-benzazepin-2-on, dessen optische Isomeren in isolierter Form oder in Form einer Mischung und dessen Additionssalze mit einer pharmazeutisch annehmbaren Säure.

5. Verbindung nach Anspruch 1, nämlich 7,8-Dimethoxy-3-{3-{N-[(5,6-dimethoxy-indan-2-yl)-methyl]-N-(methyl)-amino}-propyl}-1,3,4,5-tetrahydro-2H-3-benzazepin-2-on und dessen Additionssalze mit einer pharmazeutisch annehmbaren Säure.

6. Verbindung nach Anspruch 1, nämlich 7,8-Dimethoxy-3-{3-{N-[(5,6-dimethoxy-indan-2-yl)-methyl]-amino}-propyl}-1,3,4,5-tetrahydro-2H-3-benzazepin-2-on und dessen Additionssalze mit einer pharmazeutisch annehmbaren Säure.

7. Verfahren zur Herstellung der Verbindungen der Formel (I)**, dadurch gekennzeichnet**, daß man:
ein Amin der Formel (II): 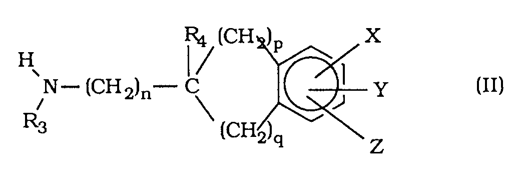 in der R₃, R₄, X, Y, Z, n, p und q die in Anspruch 1 bezüglich der Formel (I) angegebenen Bedeutungen besitzen, in Form des Racemats oder in optisch aktiver Form, wenn ein asymmetrisches Kohlenstoffatom vorliegt,
mit einer Verbindung der Formel (III): 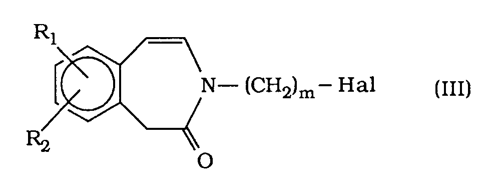 in der R₁, R₂ und m die in Anspruch 1 bezüglich der Formel (I) angegebenen Bedeutungen besitzen und Hal ein Halogenatom darstellt, kondensiert, zur Bildung einer Verbindung der Formel (IV): 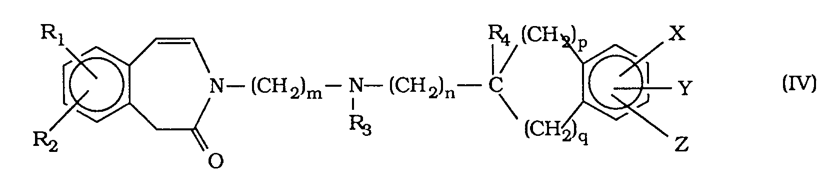 in der R₁, R₂, R₃, R₄, X, Y, Z, m, n, p und q die oben angegebenen Bedeutungen besitzen, in Form des Racemats oder in optisch aktiver Form, wenn ein asymmetrisches Kohlenstoffatom vorliegt,
welche Verbindung der Formel IV man einer Hydrierung unterzieht zur Bildung einer Verbindung der Formel (I): 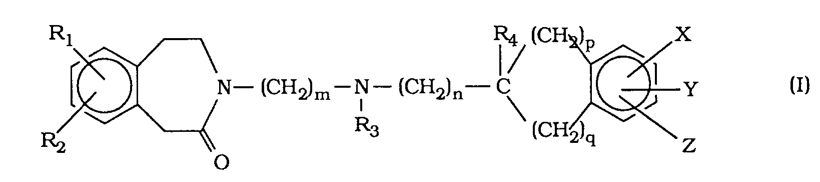 in der R₁, R₂, R₃, R₄, X, Y, Z, m, n, p und q die oben angegebenen Bedeutungen besitzen, in racemischer Form oder in optisch aktiver Form, wenn ein asymmetrisches Kohlenstoffatom existiert,
welche Verbindung der Formel (I) gewünschtenfalls:
- mit Hilfe einer klassischen Technik der Kristallisation und/oder der Chromatographie gereinigt wird und/oder
- mit einer pharmazeutisch annehmbaren Säure in ein Salz überführt wird, wobei es sich versteht, daß die Verbindungen der Formel (I), wenn sie ein asymmetrisches Kohlenstoffatom aufweisen, in optisch aktiver Form hergestellt werden können nicht nur unter Verwendung von optisch aktiven Ausgangsmaterialien, sondern auch ausgehend von den entsprechenden racemischen Verbindungen der Formel (I) durch klassische Methoden zur Trennung von optischen Isomeren.

8. Verbindung der Formel (IV): 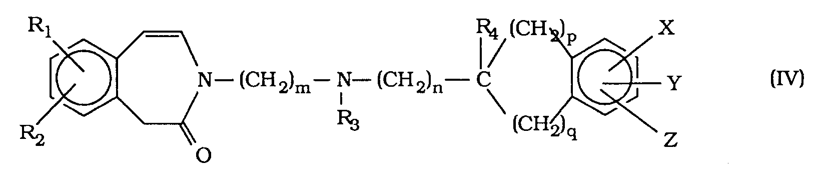 in der R₁, R₂, R₃, R₄, X, Y, Z, m, n, p und q die in Anspruch 1 bezüglich der Formel (I) angegebenen Bedeutungen besitzen,
als Ausgangsmaterialien für die Synthese der erfindungsgemäßen Verbindungen der Formel (I) nach Anspruch 1, deren eventuelle optische Isomere in isolierter Form oder in Form einer Mischung und deren Additionssalze mit einer Säure.

9. Pharmazeutische Zubereitung enthaltend als Wirkstoff mindestens eine Verbindung nach einem der Ansprüche 1 bis 6 in Kombination mit einem oder mehreren inerten, nichttoxischen und pharmazeutisch annehmbaren Trägermaterialien oder Bindemitteln.

10. Pharmazeutische Zubereitung nach Anspruch 9, geeignet zur Vorbeugung und zur Behandlung von verschiedenen klinischen Zuständen der Myokardischämie, die durch ein Ungleichgewicht zwischen der Sauerstoffversorgung und dem Sauerstoffbedarf des Myokards verursacht sind, wie der Angina pectoris, des Myokardinfarkts und damit verknüpften Rhythmusstörungen, sowie zur Vorbeugung und zur Behandlung von pathologischen Zuständen, bei denen insbesondere supra-ventrikuläre Rhythmusstörungen auftreten, und von Komplikationen von insbesondere koronaren arteriosklerotischen Vorfällen, verursacht durch Einschränkung der hämodynamischen Gefäßbelastung.

## Patentansprüche (Patentansprüche für folgende(n) Vertragsstaat(en): ES, GR)

1. Verfahren zur Herstellung der Verbindungen der allgemeinen Formel I: 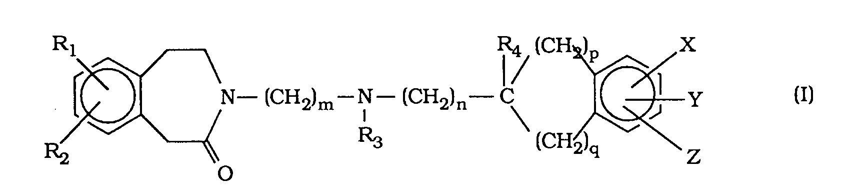 in der:
m eine ganze Zahl mit einem Wert von 2 bis 5,
n eine ganze Zahl mit einem Wert von 1 bis 6,
p und q, die gleichartig oder verschieden sein können, 0 oder eine ganze Zahl mit einem Wert von 1 bis 2,
mit der Maßgabe, daß die Summe von p + q 1 oder 2 beträgt,
R₁ und R₂, die gleichartig oder verschieden sein können, eine Gruppe ausgewählt aus:
- Wasserstoff,
- Halogen,
- Hydroxy,
- Niedrigalkoxy,
- Phenylniedrigalkoxy und
- substituiertes Phenyiniedrigalkoxy,
oder R₁ und R₂ gemeinsam, wenn sie von zwei benachbarten Kohlenstoffatomen getragen werden, eine Gruppe O-(CH₂)ᵣ-O-,
worin r eine ganze Zahl mit einem Wert von 1 oder 2 darstellt,
R₃ eine Gruppe ausgewählt aus:
- Wasserstoff,
- Niedrigalkyl,
- Niedrigalkenyl,
- Cycloalkyl,
- Cycloalkylniedrigalkyl,
- Phenylniedrigalkyl,
- substituiertes Phenylniedrigalkyl,
- -CO-R₅ oder -CO-O-R₅,
worin R₅ eine Gruppe ausgewählt aus: Wasserstoff, Niedrigalkyl, Niedrigalkenyl, Niedrigalkinyl, Phenyl, substituiertes Phenyl, Phenylniedrigalkyl, substituiertes Phenylniedrigalkyl, Niedrigcycloalkyl und Cycloalkylniedrigalkyl darstellt, und
- -CO-NR₆R₇, worin R₆ und R₇, die gleichartig oder verschieden sein können, die gleichen Bedeutungen besitzen wie die oben definierte Gruppe R₅, oder
R₆ und R₇ gemeinsam mit dem Stickstoffatom, das sie trägt, einen gesättigten Ring mit 4 bis 7 Kettengliedern,
R₄ ein Wasserstoffatom oder eine Niedrigalkylgruppe und
X, Y und Z, die gleichartig oder verschieden sein können, eine Gruppe ausgewählt aus:
- Wasserstoff,
- Halogen,
- Hydroxy,
- Niedrigalkoxy,
- substituiertes Phenylniedrigalkoxy, oder
X und Y oder Y und Z gemeinsam, wenn sie von zwei benachbarten Kohlenstoffatomen getragen werden, eine Gruppe -O-(CH₂)ᵣ-O-, in der r eine ganze Zahl mit einem Wert von 1 oder 2 darstellt, eine Gruppe -O-(CH₂)₂- oder eine Gruppe -O-CH=CH- bedeuten;
wobei es sich versteht, daß der Begriff "substituiert" bezüglich der Gruppen "Phenyl", "Phenylniedrigalkyl" und "Phenylniedrigalkoxy" bedeutet, daß diese Gruppen am Phenylkern durch einen oder mehrere Substituenten ausgewählt aus Halogenatomen und Hydroxylgruppen, Trifluormethylgruppen, Niedrigalkylgruppen und Niedrigalkoxygruppen substituiert sein können,
wobei es sich weiterhin versteht,
- daß die Begriffe "Niedrigalkyl" und "Niedrigalkoxy" für geradkettige oder verzweigte gesättigte Kohlenwasserstoffgmppen, die 1 bis 6 Kohlenstoffatome aufweisen, stehen,
- die Begriffe "Niedrigalkenyl" und "Niedrigalkinyl" für geradkettige oder verzweigte ungesättigte Gruppen, die 2 bis 6 Kohlenstoffatome aufweisen, stehen und
- der Begriff "Cycloalkyl" für einen gesättigten Kohlenwasserstoffring mit 3 bis 8 Kettengliedern steht,
von deren möglichen optischen Isomeren in isolierter Form oder in Form einer Mischung, sowie gegebenenfalls ihren Additionssalzen mit einer pharmazeutisch annehmbaren Säure, von deren möglichen optischen Isomeren sowie von deren Additionssalzen mit einer pharmazeutisch annehmbaren Säure, **dadurch gekennzeichnet**, daß man
ein Amin der Formel (II): 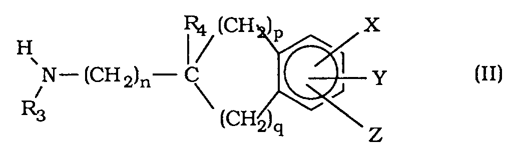 in der R₃, R₄, X, Y, Z, n, p und q die in Anspruch 1 bezüglich der Formel (I) angegebenen Bedeutungen besitzen, in Form des Racemats oder in optisch aktiver Form, wenn ein asymmetrisches Kohlenstoffatom vorliegt,
mit einer Verbindung der Formel (III): 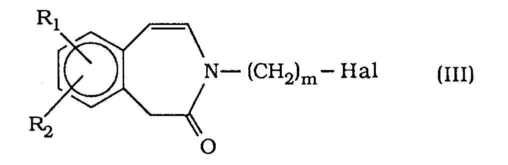 in der R₁, R₂ und m die in Anspruch 1 bezüglich der Formel (I) angegebenen Bedeutungen besitzen und Hal ein Halogenatom darstellt, kondensiert,
zur Bildung einer Verbindung der Formel (IV): 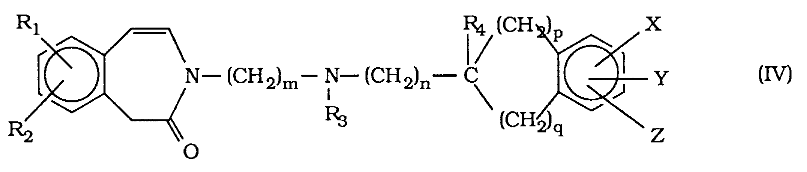 in der R₁, R₂, R₃, R₄, X, Y, Z, m, n, p und q die oben angegebenen Bedeutungen besitzen, in Form des Racemats oder in optisch aktiver Form, wenn ein asymmetrisches Kohlenstoffatom vorliegt.
welche Verbindung der Formel IV man einer Hydrierung unterzieht zur Bildung einer Verbindung der Formel (I): 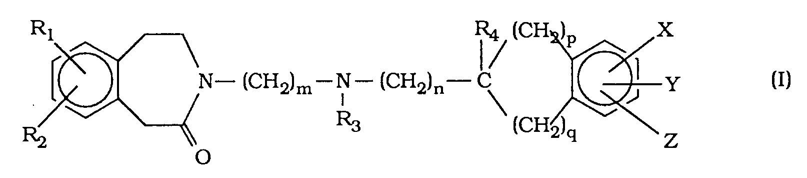 in der R₁, R₂, R₃, R₄, X, Y, Z, m, n, p und q die oben angegebenen Bedeutungen besitzen, in racemischer Form oder in optisch aktiver Form, wenn ein asymmetrisches Kohlenstoffatom existiert,
welche Verbindung der Formel (I) gewünschtenfalls:
- mit Hilfe einer klassischen Technik der Kristallisation und/oder der Chromatographie gereinigt wird und/oder
- mit einer pharmazeutisch annehmbaren Säure in ein Salz überfuhrt wird, wobei es sich versteht, daß die Verbindungen der Formel (I), wenn sie ein asymmetrisches Kohlenstoffatom aufweisen, in optisch aktiver Form hergestellt werden können nicht nur unter Verwendung von optisch aktiven Ausgangsmaterialien, sondern auch ausgehend von den entsprechenden racemischen Verbindungen der Formel (I) durch klassische Methoden zur Trennung von optischen Isomeren.
